# EUROPEAN PATENT SPECIFICATION

(11) **EP 1 613 326 B1**
(45) Date of publication and mention of the grant of the patent: **13.09.2006**
(21) Application number: 04759820.6
(22) Date of filing: 07.04.2004
(51) Int. Cl.: A61K 31/505, A61K 31/4402, A61K 31/426, A61K 31/421, C07D 239/42, C07D 213/38, C07D 277/82, C07D 263/58, A61P 3/04

(54) **(3-{3-'(2,4-BIS-TRIFLUORMETHYL-BENZYL)-(5-ETHYL-PYRIMIDIN-2-YL)-AMINO]-PROPOXY}-PHENYL)-ACETIC ACID AND RELATED COMPOUNDS AS MODULATORS OF PPARS AND METHODS OF TREATING METABOLIC DISORDERS**
(3-{3-'(2,4-BIS-TRIFLUORMETHYL-BENZYL)-(5-ETHYL-PYRIMIDIN-2-YL)-AMINO]-PROPOXY}-PHENYL)-ESSIGSÄURE UND VERWANDTE VERBINDUNGEN ALS MODULATOREN VON PPARS UND VERFAHREN ZUR BEHANDLUNG VON STOFFWECHSELSTÖRUNGEN
ACIDE(3-{3-'(2,4-BIS-TRIFLUORMETHYL-BENZYL)-(5-ETHYL-PYRIMIDIN-2-YL)-AMINO]-PROPOXY} -PHENYL)-ACETIQUE ET COMPOSES ASSOCIES EN TANT QUE MODULATEURS DE PPAR, ET PROCEDES POUR TRAITER DES TROUBLES DU METABOLISME

(30) Priority: 17.04.2003 US 464581 P
(43) Date of publication of application: 11.01.2006
(73) Proprietor: Kalypsys, Inc., San Diego, CA 92121 (US)
(72) Inventor: LIU, Kevin, Princeton, NJ08543 (US); ZHAO, Cunxiang, San Diego, CA 92130 (US)
(74) Representative: Albrecht, Thomas
(86) International application number: PCT/US2004/010970
(87) International publication number: WO 2004/093879

(56) References cited:
- WO-A-02/46176
- WO-A-97/25042

## Description

### CROSS-REFERENCE TO RELATED APPLICATIONS

This application claims the benefit of U.S. Provisional Application No. 60/464,581 filed April 17, 2003.

### FIELD OF THE INVENTION

The present invention is in the field of medicinal chemistry. More specifically, the present invention relates to aryl compounds and methods for treating various diseases by modulation of nuclear receptor mediated processes using these compounds, and in particular processes mediated by peroxisome proliferator activated receptors (PPARs).

### BACKGROUND OF THE INVENTION

Peroxisome proliferators are a structurally diverse group of compounds which, when administered to mammals, elicit dramatic increases in the size and number of hepatic and renal peroxisomes, as well as concomitant increases in the capacity of peroxisomes to metabolize fatty acids via increased expression of the enzymes required for the β-oxidation cycle (Lazarow and Fujiki, *Ann. Rev. Cell Biol.* 1:489-530 (1985); Vamecq and Draye, *Essays Biochem.* 24:1115-225 (1989); and Nelali et al., *Cancer Res.* 48:5316-5324 (1988)). Compounds that activate or otherwise interact with one or more of the PPARs have been implicated in the regulation of triglyceride and cholesterol levels in animal models. Compounds included in this group are the fibrate class of hypolipidermic drugs, herbicides, and phthalate plasticizers (Reddy and Lalwani, *Crit. Rev. Toxicol.* 12:1-58 (1983)). Peroxisome proliferation can also be elicited by dietary or physiological factors such as a high-fat diet and cold acclimatization.

Biological processes modulated by PPAR are those modulated by receptors, or receptor combinations, which are responsive to the PPAR receptor ligands. These processes include, for example, plasma lipid transport and fatty acid catabolism, regulation of insulin sensitivity and blood glucose levels, which are involved in hypoglycemia/hyperinsulinemia (resulting from, for example, abnormal pancreatic beta cell function, insulin secreting tumors and/or autoimmune hypoglycemia due to autoantibodies to insulin, the insulin receptor, or autoantibodies that are stimulatory to pancreatic beta cells), macrophage differentiation which lead to the formation of atherosclerotic plaques, inflammatory response, carcinogenesis, hyperplasia, and adipocyte differentiation.

Subtypes of PPAR include PPAR-alpha, PPAR-delta (also known as NUC1, PPAR-beta, and FAAR) and two isoforms of PPAR-gamma. These PPARs can regulate expression of target genes by binding to DNA sequence elements, termed PPAR response elements (PPRE). To date, PPRE's have been identified in the enhancers of a number of genes encoding proteins that regulate lipid metabolism suggesting that PPARs play a pivotal role in the adipogenic signaling cascade and lipid homeostasis (H. Keller and W. Wahli, *Trends Endoodn. Met.* 291-296, 4 (1993)).

Insight into the mechanism whereby peroxisome proliferators exert their pleiotropic effects was provided by the identification of a member of the nuclear hormone receptor superfamily activated by these chemicals (Isseman and Green, Nature 347-645-650 (1990)). The receptor, termed PPAR-alpha (or alternatively, PPARα), was subsequently shown to be activated by a variety of medium and long-chain fatty acids and to stimulate expression of the genes encoding rat acyl-CoA oxidase and hydratase-dehydrogenase (enzymes required for peroxisomal β-oxidation), as well as rabbit cytochrome P450 4A6, a fatty acid ω-hydroxylase (Gottlicher et al., Proc. Natl. Acad. Sci. USA 89:4653-4657 (1992); Tugwood et al., EMBO J 11:433-439 (1992); Bardot et al., Biochem. Biophys. Res. Comm. 192:37-45 (1993); Muerhoff et al., J Biol. Chem. 267:19051-19053 (1992); and Marcus et al., Proc. Natl. Acad Sci. USA 90(12):5723-5727 (1993).

Activators of the nuclear receptor PPAR-gamma (or alternatively, PPARγ), for example troglitazone, have been clinically shown to enhance insulin-action, to reduce serum glucose and to have small but significant effects on reducing serum triglyceride levels in patients with Type 2 diabetes. See, for example, D. E. Kelly et al., *Curr. Opin. Endocrinol. Diabetes,* 90-96, 5 (2), (1998); M. D. Johnson et al., *Ann. Pharmacother.,* 337-348, 32 (3), (1997); and M. Leutenegger et al., *Curr. Ther. Res.,* 403-416, 58 (7), (1997).

PPAR-delta (or alternatively, PPARδ) is broadly expressed in the body and has been shown to be a valuable molecular target for treatment of dyslipedimia and other diseases. For example, in a recent study in insulin-resistant obese rhesus monkeys, a potent and selective PPAR-delta compound was shown to decrease VLDL and increase HDL in a dose response manner (Oliver et al., *Proc. Natl. Acad. Sci. U. S. A.*98*:* 5305, 2001).

Because there are three isoforms of PPAR and all of them have been shown to play important roles in energy homeostasis and other important biological processes in human body and have been shown to be important molecular targets for treatment of metabolic and other diseases (see Willson, et al. J. Med. Chem. 43: 527-550 (2000)), it is desired in the art to identify compounds which are capable of selectively interacting with only one of the PPAR isoforms or compounds which are capable of interacting with multiple PPAR isoforms. Such compounds would find a wide variety of uses, such as, for example, in the treatment or prevention of obesity, for the treatment or prevention of diabetes, dyslipidemia, metabolic syndrome X and other uses.

### SUMMARY OF THE INVENTION

Described herein are compounds capable of modulating nuclear receptor processes mediated by peroxisome proliferator activated receptors (PPARs), and methods for utilizing such modulation in the treatment of metabolic diseases, conditions, and disorders. Also described are carbocyclic aryl-derived compounds that mediate and/or inhibit the activity of peroxisome proliferator activated receptors (PPARs), and pharmaceutical compositions containing such compounds. Also described are therapeutic or prophylactic use of such compounds and compositions, and methods of treating metabolic diseases, conditions, and disorders, by administering effective amounts of such compounds.

In one aspect are compounds having the structure of Formula I: 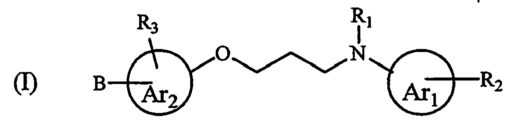 wherein
Ar₁ is selected from the group consisting of a monocyclic heteroaromatic ring structure and a bicyclic heteroaromatic ring structure;
Ar₂ is selected from the group consisting of a monocyclic, a bicyclic, and a tricyclic carbocyclic aryl ring structure
R₁ is selected from the group consisting of
alkyl, optionally substituted with a substituent selected from the group consisting of hydrogen, lower alkyl, optionally substituted carbocyclic or heterocyclic ring, halogen, perhaloalkyl, hydroxy, alkoxy, nitro, and amino;
a five-membered or six-membered heteroaryl ring or a six-membered aryl ring, optionally substituted with one or more substituents selected from the group consisting of optionally substituted C₁-C₈ straight-chain, branched, or cyclic saturated or unsaturated alkyl; an alkoxy; cyano; nitro; an amino; an amido; perhaloalkyl; and halogen;
R₂ is selected from the group consisting of
hydrogen;
alkyl, optionally substituted with a substituent selected from the group consisting of hydrogen, lower alkyl, optionally substituted carbocyclic or heterocyclic ring, halogen, perhaloalkyl, hydroxy, alkoxy, nitro, and amino;
a five-membered or six-membered heteroaryl ring or a six-membered aryl ring, optionally substituted with one or more substituents selected from the group consisting of optionally substituted C₁-C₈ straight-chain, branched, or cyclic saturated or unsaturated alkyl; an alkoxy; halogen; and perhaloalkyl;
cyano; nitro; an amino; an amido; perhaloalkyl; and halogen;
R₃ is selected from the group consisting of hydrogen; alkyl, optionally substituted with a substituent selected from the group consisting of hydrogen, lower alkyl, optionally substituted carbocyclic or heterocyclic ring; hydroxy; halogen; amino; nitro; and cyano; and
B is a five-membered or six-membered heteroaryl ring, or -(CH₂)ⱼ-C(O)OR₄, wherein j is
0 or 1 when Ar₂ is a bicyclic or tricyclic carbocyclic ring structure and j is 1 when Ar₂ is a monocyclic carbocyclic ring structure; and
R₄ is selected from the group consisting of
hydrogen;
alkyl, optionally substituted with a substituent selected from the group consisting of hydrogen, lower alkyl, optionally substituted carbocyclic or heterocyclic ring;
a five-membered or six-membered heteroaryl ring or a six-membered aryl ring, optionally substituted with one or more substituents selected from the group consisting of optionally substituted C₁-C₈ straight-chain, branched,
or cyclic saturated or unsaturated alkyl;
or a pharmaceutically acceptable N-oxide, pharmaceutically acceptable prodrug, pharmaceutically active metabolite, pharmaceutically acceptable salt, pharmaceutically acceptable ester, pharmaceutically acceptable amide, or pharmaceutically acceptable solvate thereof.

In one embodiment of this aspect, Ar₂ is selected from the group consisting of phenyl, naphthyl, anthracene, and phenanthrene. In a further embodiment of this aspect, Ar₂ is phenyl. In yet a further embodiment of this aspect, the compound has the structure of Formula (II):

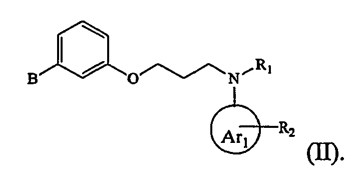

In an alternative further embodiment of this aspect, Ar₂ is naphthyl. In a further embodiment of this aspect, Ar₂ is optionally either naphthyl or the compound has the structure of Formula (II); for convenience, this embodiment that encompasses these two alternative options for Ar₂ will be called Embodiment 2. In a further embodiment of Embodiment 2, R₁ is alkyl, optionally substituted with one or more optionally substituted carbocyclic or heterocyclic rings. In yet a further embodiment of Embodiment 2, the alkyl is a lower alkyl. In yet a further embodiment of Embodiment 2, the lower alkyl is selected from the group consisting of methyl, ethyl, n-propyl, isopropyl, n-butyl, tert-butyl, and sec-butyl.

In an alternative embodiment of Embodiment 2, R₁ is alkyl substituted with an optionally substituted phenyl (i.e., the carbocyclic ring is phenyl). In yet a further embodiment, the phenyl is optionally substituted with one or more substituents selected from the group consisting of lower alkyl, halogen, perhaloalkyl, hydroxy, alkoxy, nitro, and amino. In still a further embodiment, the substituent is perhaloalkyl. And, in yet a further embodiment, the perhaloalkyl is trifluoromethyl.

In another embodiment of the compounds having the structure of Formula (I), R₁ is 4-bis(trifluoromethyl)phenylmethyl.

In yet another embodiment of the compounds having the structure of Formula (I), Ar₁ is selected from the group consisting of furan, thiophene, pyrrole, pyrroline, pyrrolidine, oxazole, thiazole, imidazole, imidazoline, imidazolidine, pyrazole, pyrazoline, pyrazolidine, isoxazole, isothiazole, triazole, tetrazole, thiadiazole, pyran, pyridine, piperidine, morpholine, thiomorpholine, pyridazine, pyrimidine, pyrazine, piperazine, triazine, 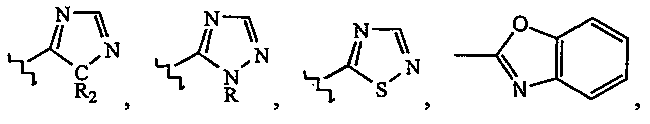 and 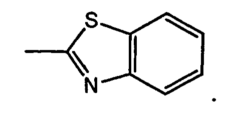 In yet a further embodiment, Ar₁ is pyridine, pyrimidine, 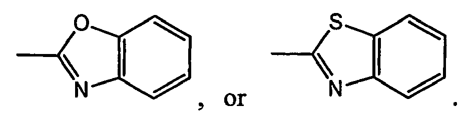 In still a further embodiment, Ar₁ is pyrimidine.

In another embodiment of Embodiment 2 (see above), R₂ is optionally substituted alkyl. In yet a further embodiment, the alkyl is a lower alkyl. In still a further embodiment, the lower alkyl is selected from the group consisting of methyl, ethyl, n-propyl, isopropyl, n-butyl, tert-butyl, and sec-butyl. And in yet a further embodiment, R₂ is ethyl.

In another embodiment of the compounds having the structure of Formula (I), R₃ is hydrogen, halogen or optionally substituted alkyl. In one embodiment R₃ is hydrogen. In an alternative embodiment, R₃ is an optionally substituted alkyl that is an optionally substituted lower alkyl. In still a further embodiment, the optionally substituted lower alkyl is selected from the group consisting of methyl, ethyl, n-propyl, isopropyl, n-butyl, tert-butyl, and sec-butyl. And in still a further embodiment, R₃ is methyl.

In a set of alternative embodiments to the compounds having the structure of Formula (I), (a) B and the propyloxy substituents on Ar₂ are ortho to each other; (b) B and the propyloxy substituents on Ar₂ are meta to each other; and (c) B and the propyloxy substituents on Ar₂ are para to each other.

In a further embodiment of the compounds having the structure of Formula (I), B is a heteroaryl ring selected from the group consisting of furan, thiophene, pyrrole, pyrroline, pyrrolidine, oxazole, thiazole, imidazole, imidazoline, imidazolidine, pyrazole, pyrazoline, pyrazolidine, isoxazole, isothiazole, triazole, tetrazole, thiadiazole, pyran, pyridine, piperidine, morpholine, thiomorpholine, pyridazine, pyrimidine, pyrazine, piperazine, triazine,

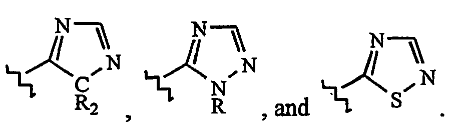

In a further embodiment, B is a tetrazole.

In another embodiment of the compounds having the structure of Formula (I), B is -(CH₂)ⱼ-C(O)OR₄. In a further embodiment, R₄ is hydrogen or optionally substituted alkyl. In one embodiment of this option, R₄ is hydrogen. In an alternative embodiment, R₄ is alkyl. In a further embodiment of this latter embodiment, the alkyl is a lower alkyl. In yet a further embodiment, the lower alkyl is selected from the group consisting of methyl, ethyl, n-propyl, isopropyl, n-butyl, tert-butyl, and sec-butyl.

In another embodiment of Embodiment 2 (see above), Ar₁ is selected from the group consisting of furan, thiophene, pyrrole, pyrroline, pyrrolidine, oxazole, thiazole, imidazole, imidazoline, imidazolidine, pyrazole, pyrazoline, pyrazolidine, isoxazole, isothiazole, triazole, tetrazole, thiadiazole, pyran, pyridine, piperidine, morpholine, thiomorpholine, pyridazine, pyrimidine, pyrazine, piperazine, triazine, 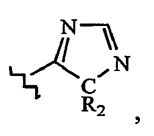 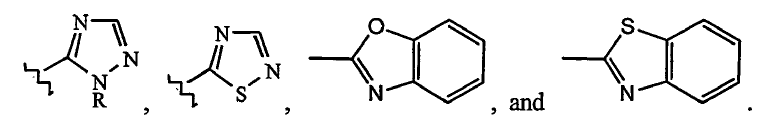 In yet a further embodiment, Ar₁ is pyridine, pyrimidine, 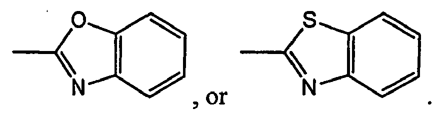 In still a further embodiment, Ar₁ is pyrimidine.

In another embodiment of Embodiment 2 (see above), B is a heteroaryl ring selected from the group consisting of furan, thiophene, pyrrole, pyrroline, pyrrolidine, oxazole, thiazole, imidazole, imidazoline, imidazolidine, pyrazole, pyrazoline, pyrazolidine, isoxazole, isothiazole, triazole, tetrazole, thiadiazole, pyran, pyridine, piperidine, morpholine, thiomorpholine, pyridazine, pyrimidine, pyrazine, piperazine, triazine, 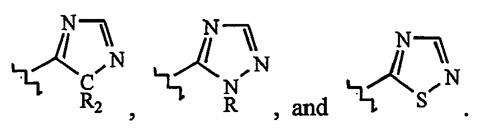 In another embodiment, B is a tetrazole.

In another embodiment of Embodiment 2, B is -(CH₂)ⱼ-C(O)OR₄. In a further embodiment, R₄ is hydrogen or optionally substituted alkyl. In one alternative of this embodiment, R₄ is hydrogen. In an alternative embodiment of this embodiment, R₄ is an optionally substituted alkyl. In a further embodiment of this latter alternative, the alkyl is a lower alkyl. In still a further embodiment, the lower alkyl is selected from the group consisting of methyl, ethyl, n-propyl, isopropyl, n-butyl, tert-butyl, and sec-butyl.

In another embodiment of the compounds having the structure of Formula (I), the compound is selected from the group consisting of: 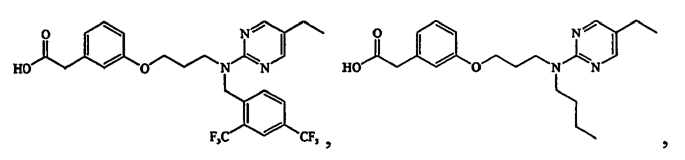 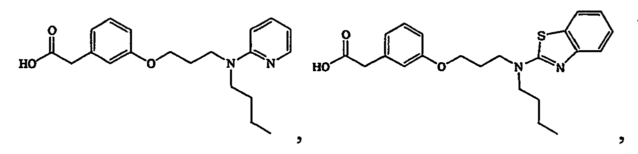 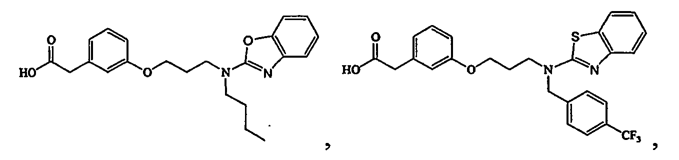 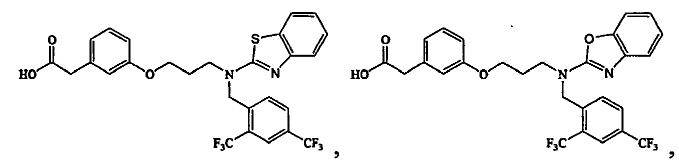 and 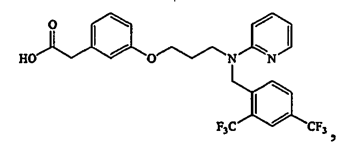 or a pharmaceutically acceptable N-oxide, pharmaceutically acceptable prodrug, pharmaceutically active metabolite, pharmaceutically acceptable salt, pharmaceutically acceptable ester, pharmaceutically acceptable amide, or pharmaceutically acceptable solvate thereof.
In another embodiment of the compounds having the structure of Formula (I), the compound is selected from the group consisting of: 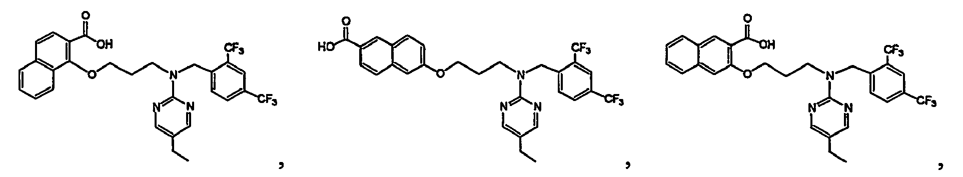 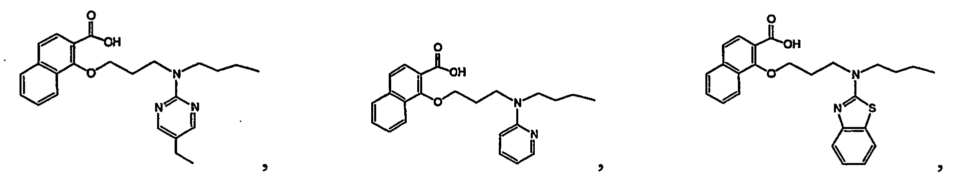 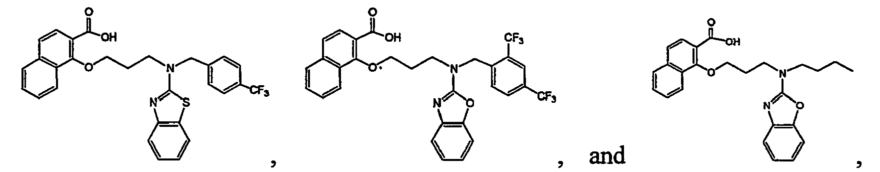 or a pharmaceutically acceptable N-oxide, pharmaceutically acceptable prodrug, pharmaceutically active metabolite, pharmaceutically acceptable salt, pharmaceutically acceptable ester, pharmaceutically acceptable amide, or pharmaceutically acceptable solvate thereof.

A compound having the structure of Formula III:

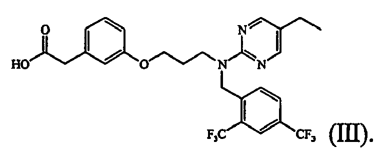

Another aspect presented herein is method of modulating a peroxisome proliferator-activated receptor (PPAR) function comprising contacting said PPAR with a compound having the structure of Formula (I) and monitoring a change in cell phenotype, cell proliferation, activity of said PPAR, or binding of said PPAR with a natural binding partner. In a further embodiment of this aspect, the PPAR is selected from the group consisting of PPARα, PPARδ, and PPARγ.

Another aspect presented herein is a method of inhibiting the formation of adipocytes in a mammal comprising administering a therapeutically effective amount of a compound having the structure of Formula (I) to said mammal.

In one embodiment of this aspect, the mammal is administered a therapeutically effective amount of a compound having the structure of Formula (1), wherein Ar₂ is phenyl. In another embodiment of this aspect, the mammal is administered a therapeutically effective amount of a compound having the structure of Formula (I), wherein Ar₂ is napthyl. In another embodiment of this aspect, the mammal is administered a therapeutically effective amount of a compound having the structure of Formula (II).

Another aspect presented herein is a method of inhibiting the formation of adipocytes in a mammal comprising administering a therapeutically effective amount of a compound having the structure of Formula (III) to said mammal.

Another aspect presented herein is a method of treating a disease comprising identifying a patient in need thereof, and administering a therapeutically effective amount of a compound having the structure of Formula (I) to said patient. In a further embodiment, the disease is a PPAR-modulated disease. In a further or alternative embodiment, the disease is a metabolic disorder or condition. In an further or alternative embodiment, the disease is selected from the group consisting of obesity, diabetes, hyperinsulinemia, metabolic syndrome X, polycystic ovary syndrome, climacteric, disorders associated with oxidative stress, inflammatory response to tissue injury, pathogenesis of emphysema, ischemia-associated organ injury, doxorubicin-induced cardiac injury, drug-induced hepatotoxicity, atherosclerosis, and hypertoxic lung injury. In one such case, the mammal is administered a therapeutically effective amount of a compound having the structure of Formula (I), wherein Ar₂ is phenyl. In another such case, the mammal is administered a therapeutically effective amount of a compound having the structure of Formula (I), wherein Ar₂ is napthyl. In yet another such case, the mammal is administered a therapeutically effective amount of a compound having the structure of Formula (II).

In another aspect presented herein is a method of treating a PPAR-modulated disease comprising identifying a patient in need thereof, and administering a therapeutically effective amount of a compound having the structure of Formula (III) to said patient.

Another aspect presented herein is a pharmaceutical composition comprising a compound having the structure of Formula (I) and a pharmaceutically acceptable diluent, excipient, or carrier. In, one embodiment of this aspect, Ar₂ is aryl. In another embodiment of this aspect, Ar₂ is naphthyl. In yet another embodiment of this aspect, the compound has the structure of Formula (II). In another aspect presented herein is a pharmaceutical composition comprising a compound having the structure of Formula (III) and a pharmaceutically acceptable diluent, excipient, or carrier.

### DETAILED DESCRIPTION OF THE INVENTION

The present invention discloses that a substituted carbocyclic aryl moiety linked to an optionally substituted heterocyclic moiety by an -O-(CH₂)₃-NR₁- group can modulate at least one peroxisome proliferator-activated receptor (PPAR) function, and can confer additionally selective activation of hPPAR-gamma. The carbocyclic aryl moiety in one embodiment is a naphthyl group and in another embodiment is a phenyl group. In one particular embodiment, when the carbocyclic aryl group is a phenyl group, a -CH₂C(O)OR₄ substituent and the linking -O-(CH₂)₃-NR₁- group are oriented meta to each other on the phenyl ring.

Compounds described herein may be activating both PPAR-delta and PPAR-gamma or PPAR-alpha and PPAR-delta, or all three PPAR subtypes, or selectively activating predominantly hPPAR-gamma, hPPAR-alpha or hPPAR-delta.

The present invention relates to a method of modulating at least one peroxisome proliferator-activated receptor (PPAR) function comprising the step of contacting the PPAR with a compound of Formula I, as described herein. The change in cell phenotype, cell proliferation, activity of the PPAR, expression of the PPAR or binding of the PPAR with a natural binding partner may be monitored. Such methods may be modes of treatment of disease, biological assays, cellular assays, biochemical assays, or the like.

The present invention describes methods of treating a disease comprising identifying a patient in need thereof, and administering a therapeutically effective amount of a compound of Formula I, as described herein, to a patient. Thus, in certain embodiments, the disease to be treated by the methods of the present invention is selected from the group consisting of obesity, diabetes, hyperinsulinemia, metabolic syndrome X, polycystic ovary syndrome, climacteric, disorders associated with oxidative stress, inflammatory response to tissue injury, pathogenesis of emphysema, ischemia-associated organ injury, doxorubicin-induced cardiac injury, drug-induced hepatotoxicity, atherosclerosis, and hypertoxic lung injury.

### I. CHEMICAL TERMINOLOGY

An "acetyl" group refers to a -C(=O)CH₃, group.

The term "acyl" includes alkyl, aryl, or heteroaryl substituents attached to a compound via a carbonyl functionality (e.g., -C(O)-alkyl, -C(O)-aryl, etc.).

An "alkoxy" group refers to a RO- group, where R is as defined herein.

An "alkoxyalkoxy" group refers to a ROR'O- group, where R is as defined herein.

An "alkoxyalkyl" group refers to a R'OR- group, where R and R' are as defined herein.

As used herein, the term "alkyl" refers to an aliphatic hydrocarbon group. The alkyl moiety may be a "saturated alkyl" group, which means that it does not contain any alkene or alkyne moieties. The alkyl moiety may also be an "unsaturated alkyl" moiety, which means that it contains at least one alkene or alkyne moiety. An "alkene" moiety refers to a group consisting of at least two carbon atoms and at least one carbon-carbon double bond, and an "alkyne" moiety refers to a group consisting of at least two carbon atoms and at least one carbon-carbon triple bond. The alkyl moiety, whether saturated or unsaturated, may be branched, straight chain, or cyclic.

The "alkyl" moiety may have 1 to 40 carbon atoms (whenever it appears herein, a numerical range such as "1 to 40" refers to each integer in the given range; *e.g*., "1 to 40 carbon atoms" means that the alkyl group may consist of 1 carbon atom, 2 carbon atoms, 3 carbon atoms, *etc.*, up to and including 40 carbon atoms, although the present definition also covers the occurrence of the term "alkyl" where no numerical range is designated). The alkyl group may be a "medium alkyl" having 1 to 20 carbon atoms. The alkyl group could also be a "lower alkyl" having 1 to 5 carbon atoms. The alkyl group of the compounds of the invention may be designated as "C₁-C₄ alkyl" or similar designations. By way of example only, "C₁-C₄ alkyl" indicates that there are one to four carbon atoms in the alkyl chain, i.e., the alkyl chain is selected from the group consisting of methyl, ethly, propyl; iso-propyl, n-butyl, iso-butyl, sec-butyl, and t-butyl. Typical alkyl groups include, but are in no way limited to, methyl, ethyl, propyl, isopropyl, butyl, isobutyl, tertiary butyl, pentyl, hexyl, ethenyl, propenyl, butenyl, cyclopropyl, cyclobutyl, cyclopentyl, cyclohexyl, and the like. An alkyl group may be optionally substituted.

The term "alkylamino" refers to the -NRR' group, where R and R' are as defined herein. R and R', taken together, can optionally form a cyclic ring system.

The term "alkylene" refers to an alkyl group that is substituted at two ends (i.e., a diradical). Thus, methylene (-CH₂-) ethylene (-CH₂CH₂-), and propylene (-CH₂CH₂CH₂-) are examples of alkylene groups. Similarly, "alkenylene" and "alkynylene" groups refer to diradical alkene and alkyne moieties, respectively. An alkylene group may be optionally substituted.

An "amide" is a chemical moiety with formula -C(O)NHR or -NHC(O)R, where R is optionally substituted and is selected from the group consisting of alkyl, cycloalkyl, aryl, heteroaryl (bonded through a ring carbon) and heteroalicyclic (bonded through a ring carbon). An amide may be an amino acid or a peptide molecule attached to a molecule of the present invention, thereby forming a prodrug. Any amine, hydroxy, or carboxyl side chain on the compounds of the present invention can be amidified. The procedures and specific groups to be used to achieve makes such amides are known to those of skill in the art and can readily be found in reference sources such as Greene and Wuts, Protective Groups in Organic Synthesis, 3^{rd} Ed., John Wiley & Sons, New York, NY, 1999, which is incorporated herein by reference in its entirety.

A "C-amido" group refers to a -C(=O)-NR₂ group with R as defined herein.

An "N-amido" group refers to a RC(=O)NH- group, with R as defined herein.

The term "aromatic" or "aryl" refers to an aromatic group which has at least one ring having a conjugated pi electron system and includes both carbocyclic aryl (e.g., phenyl) and heterocyclic aryl (or "heteroaryl" or "heteroaromatic") groups (e.g., pyridine). The term includes monocyclic or fused-ring polycyclic (i.e., rings which share adjacent pairs of carbon atoms) groups. The term "carbocyclic" refers to a compound which contains one or more covalently closed ring structures, and that the atoms forming the backbone of the ring are all carbon atoms. The term thus distinguishes carbocyclic from heterocyclic rings in which the ring backbone contains at least one atom which is different from carbon. An aromatic or aryl group may be optionally substituted.

An "O-carbamyl" group refers to a -OC(=O)-NR, group-with R as defined herein.

An "N-carbamyl" group refers to a ROC(=O)NH- group, with R as defined herein.

An "O-carboxy" group refers to a RC(=O)O- group, where R is as defined herein.

A "C-carboxy" group refers to a -C(=O)OR groups where R is as defined herein.

A "cyano" group refers to a -CN group.

The term "cycloalkyl" refers to a monocyclic or polycyclic radical which contains only carbon and hydrogen, and may be saturated, partially unsaturated, or fully unsaturated. A cycloalkyl group may be optionally substituted. Preferred cycloalkyl groups include groups having from three to twelve ring atoms, more preferably from 5 to 10 ring atoms. Illustrative examples of cycloalkyl groups include the following moieties: 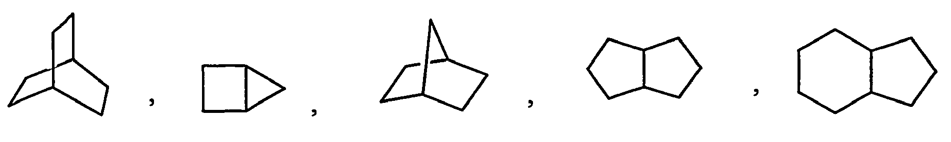 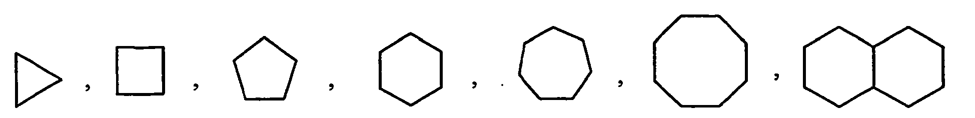 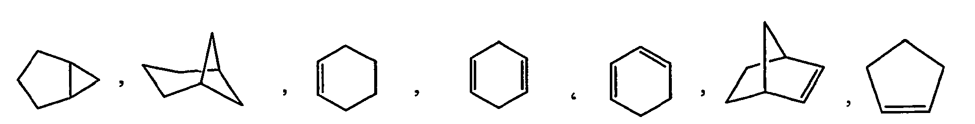 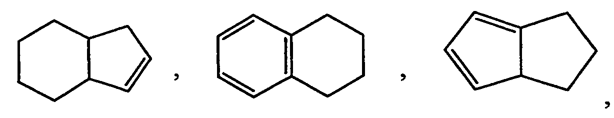 and the like.

The term "ester" refers to a chemical moiety with formula -COOR, where R is optionally substituted and is selected from the group consisting of alkyl, cycloalkyl, aryl, heteroaryl (bonded through a ring carbon) and heteroalicyclic (bonded through a ring carbon). Any amine, hydroxy, or carboxyl side chain on the compounds of the present invention can be esterified. The procedures and specific groups to be used to achieve makes such esters are known to those of skill in the art and can readily be found in reference sources such as Greene and Wuts, Protective Groups in Organic Synthesis, 3^{rd} Ed., John Wiley & Sons, New York, NY, 1999, which is incorporated herein by reference in its entirety.

The term "halo" or, alternatively, "halogen" means fluoro, chloro, bromo or iodo. Preferred halo groups are fluoro, chloro and bromo.

The terms "haloalkyl," "haloalkenyl," "haloalkynyl" and "haloalkoxy" include alkyl, alkenyl, alkynyl and alkoxy structures, that are substituted with one or more halo groups or with combinations thereof. The terms "fluoroalkyl" and "fluoroalkoxy" include haloalkyl and haloalkoxy groups, respectively, in which the halo is fluorine.

The terms "heteroalkyl" "heteroalkenyl" and "heteroalkynyl" include optionally substituted alkyl, alkenyl and alkynyl radicals and which have one or more skeletal chain atoms selected from an atom other that carbon, e.g., oxygen, nitrogen, sulfur, phosphorus or combinations thereof.

The terms "heteroaryl" or, alternatively, "heteroaromatic" refers to an aryl group that includes one or more ring heteroatoms selected from nitrogen, oxygen and sulfur. A heteroaryl group may be optionally substituted. An N-containing "heteroaromatic" or "heteroaryl" moiety refers to an aromatic group in which at least one of the skeletal atoms of the ring is a nitrogen atom. The polycyclic heteroaryl group may be fused or non-fused. Illustrative examples of aryl groups include the following moieties: 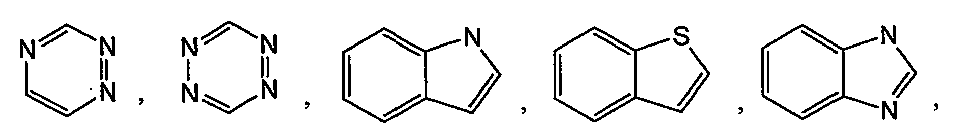 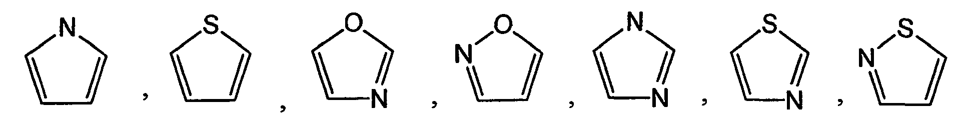 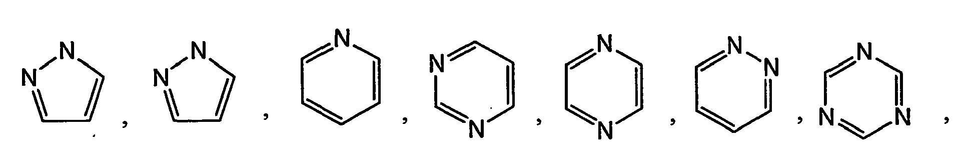 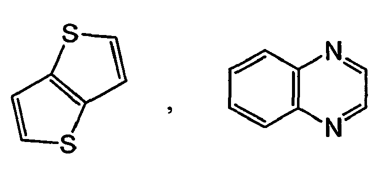 and the like.

The term "heterocycle" refers to heteroaromatic and heteralicyclic groups containing one to four heteroatoms each selected from O, S and N, wherein each heterocyclic group has from 4 to 10 atoms in its ring system, and with the proviso that the ring of said group does not contain two adjacent O or S atoms. Non-aromatic heterocyclic groups include groups having only 4 atoms in their ring system, but aromatic heterocyclic groups must have at least 5 atoms in their ring system. The heterocyclic groups include benzo-fused ring systems. An example of a 4-membered heterocyclic group is azetidinyl (derived from azetidine). An example of a 5-membered heterocyclic group is thiazolyl. An example of a 6-membered heterocyclic group is pyridyl, and an example of a 10-membered heterocyclic group is quinolinyl. Examples of non-aromatic heterocyclic groups are pyrrolidinyl, tetrahydrofuranyl, dihydrofuranyl, tetrahydrothienyl, tetrahydropyranyl, dihydropyranyl, tetrahydrothiopyranyl, piperidino, morpholino, thiomorpholino, thioxanyl, piperazinyl, azetidinyl, oxetanyl, thietanyl, homopiperidinyl, oxepanyl, thiepanyl, oxazepinyl, diazepinyl, thiazepinyl, 1,2,3,6-tetrahydropyridinyl, 2-pyrrolinyl, 3-pyrrolinyl, indolinyl, 2H-pyranyl, 4H-pyranyl, dioxanyl, 1,3-dioxolanyl, pyrazolinyl, dithianyl, dithiolanyl, dihydropyranyl, dihydrothienyl, dihydrofuranyl, pyrazolidinyl, imidazolinyl, imidazolidinyl, 3-azabicyclo[3.1.0]hexanyl, 3-azabicyclo[4.1.0]heptanyl, 3H-indolyl and quinolizinyl. Examples of aromatic heterocyclic groups are pyridinyl, imidazolyl, pyrimidinyl, pyrazolyl, triazolyl, pyrazinyl, tetrazolyl, furyl, thienyl, isoxazolyl, thiazolyl, oxazolyl, isothiazolyl, pyrrolyl, quinolinyl, isoquinolinyl, indolyl, benzimidazolyl, benzofuranyl, cinnolinyl, indazolyl, indolizinyl, phthalazinyl, pyridazinyl, triazinyl, isoindolyl, pteridinyl, purinyl, oxadiazolyl, thiadiazolyl, furazanyl, benzofurazanyl, benzothiophenyl, benzothiazolyl, benzoxazolyl, quinazolinyl, quinoxalinyl, naphthyridinyl, and furopyridinyl. The foregoing groups, as derived from the groups listed above, may be C-attached or N-attached where such is possible. For instance, a group derived from pyrrole may be pyrrol-1-yl (N-attached) or pyrrol-3-yl (C-attached). Further, a group derived from imidazole may be imidazol-1-yl or imidazol-3-yl (both N-attached) or imidazol-2-yl, imidazol-4-yl or imidazol-5-yl (all C-attached). The heterocyclic groups include benzo-fused ring systems and ring systems substituted with one or two oxo (=O) moieties such as pyrrolidin-2-one. A heterocycle group may be optionally substituted.

A "heteroalicyclic" group refers to a cycloalkyl group that includes at least one heteroatom selected from nitrogen, oxygen and sulfur. The radicals may be fused with an aryl or heteroaryl. Illustrative examples of heterocycloalkyl groups include: 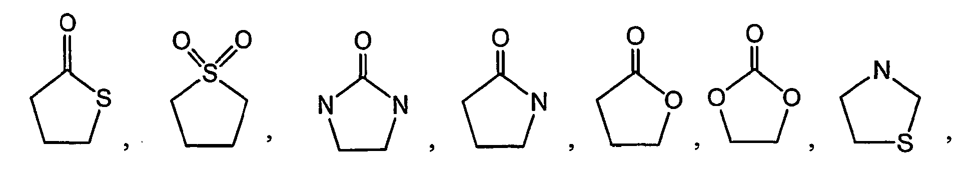 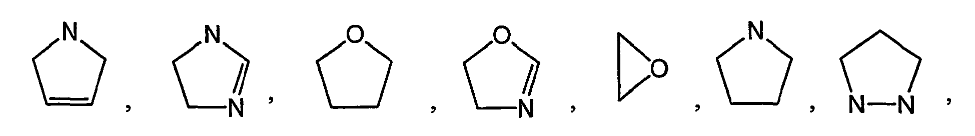 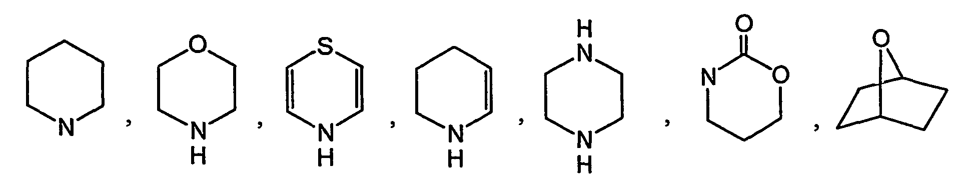 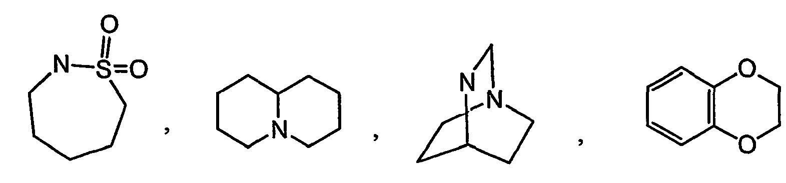 and the like.

The term "membered ring" can embrace any cyclic structure. The term "membered" is meant to denote the number of skeletal atoms that constitute the ring. Thus, for example, cyclohexyl, pyridine, pyran and thiopyran are 6-membered rings and cyclopentyl, pyrrole, furan, and thiophene are 5-membered rings.

An "isocyanato" group refers to a -NCO group.

An "isothiocyanato" group refers to a -NCS group.

A "mercaptoalkyl" group refers to a R'SR- group, where R and R'are as defined herein.

A "mercaptomercaptyl" group refers to a RSR'S- group, where R is as defined herein.

A "mercaptyl" group refers to a RS- group, where R is as defined herein.

The terms "nucleophile" and "electrophile" as used herein have their usual meanings familiar to synthetic and/or physical organic chemistry. Carbon electrophiles typically comprise one or more alkyl, alkenyl, alkynyl or aromatic (sp³, sp², or sp hybridized) carbon atoms substituted with any atom or group having a Pauling electronegativity greater than that of carbon itself. Examples of carbon electrophiles include but are not limited to carbonyls (aldehydes, ketones, esters, amides), oximes, hydrazones, epoxides, aziridines, alkyl-, alkenyl-, and aryl halides, acyls, sulfonates (aryl, alkyl and the like). Other examples of carbon electrophiles include unsaturated carbon atoms electronically conjugated with electron withdrawing groups, examples being the 6-carbon in a alpha-unsaturated ketones or carbon atoms in fluorine substituted aryl groups. Methods of generating carbon electrophiles, especially in ways which yield precisely controlled products, are known to those skilled in the art of organic synthesis.

The term "perhaloalkyl" refers to an alkyl group where all of the hydrogen atoms are replaced by halogen atoms.

The substituent R or R' appearing by itself and without a number designation refers to an optionally substituted substituent selected from the group consisting of alkyl, cycloalkyl, aryl, heteroaryl (bonded through a ring carbon) and heteroalicyclic (bonded through a ring carbon).

A "sulfinyl" group refers to a -S(=O)-R group, with R as defined herein.

A "N-sulfonamido" group refers to a RS(=O)₂NH- group with R as defined herein.

A "S-sulfonamido" group refers to a -S(=O)₂NR, group, with R as defined herein.

An "N-thiocarbamyl" group refers to an ROC(=S)NH- group, with R as defined herein.

An "O-thiocarbamyl" group refers to a -OC(=S)-NR, group with R as defined herein.

A "thiocyanato" group refers to a -CNS group.

A "trihalomethanesulfonarnido" group refers to a X₃CS(=O)₂NR- group with X and R as defined herein.

A "trihalomethanesulfonyl" group refers to a X₃CS(=O)₂- group where X is a halogen.

Unless otherwise indicated, when a substituent is deemed to be "optionally substituted," it is meant that the substituent is a group that may be substituted with one or more group(s) individually and independently selected from alkyl, perfluoroalkyl, perfluoroalkoxy, cycloalkyl, aryl, heteroaryl, heteroalicyclic, hydroxy, alkoxy, aryloxy, mercapto, alkylthio, arylthio, cyano, halo, carbonyl, thiocarbonyl, O-carbamyl, N-carbamyl, O-thiocarbamyl, N-thiocarbamyl, C-amido, N-amido, S-sulfonamido, N-sulfonamido, C-carboxy, O-carboxy, isocyanato, thiocyanato, isothiocyanato, nitro, silyl, trihalomethanesulfonyl, and amino, including mono- and di-substituted amino groups, and the protected derivatives thereof. The protecting groups that may form the protective derivatives of the above substituents are known to those of skill in the art and may be found in references such as Greene and Wuts, above.

Molecular embodiments of the present invention may possess one or more chiral centers and each center may exist in the R or S configuration. The present invention includes all diastereomeric, enantiomeric, and epimeric forms as well as the appropriate mixtures thereof. Stereoisomers may be obtained, if desired, by methods known in the art as, for example, the separation of stereoisomers by chiral chromatographic columns. Additionally, the compounds of the present invention may exist as geometric isomers. The present invention includes all cis, trans, syn, anti, entgegen (E), and zusammen (Z) isomers as well as the appropriate mixtures thereof.

In some situations, compounds may exist as tautomers. All tautomers are included within Formula I and are provided by this invention.

In addition, the compounds of the present invention can exist in unsolvated as well as solvated forms with pharmaceutically acceptable solvents such as water, ethanol, and the like. In general, the solvated forms are considered equivalent to the unsolvated forms for the purposes of the present invention.

### II. METHODS OF MODULATING PROTEIN FUNCTION

In another aspect, the present invention relates to a method of modulating at least one peroxisome proliferator-activated receptor (PPAR) function comprising the step of contacting the PPAR with a compound of Formula I, as described herein. The change in cell phenotype, cell proliferation, activity of the PPAR, or binding of the PPAR with a natural binding partner may be monitored. Such methods may be modes of treatment of disease, biological assays, cellular assays, biochemical assays, or the like. In certain embodiments, the PPAR may be selected from the group consisting of PPARα, PPARδ, and PPARγ.

The term "activate" refers to increasing the cellular function of a PPAR. The term "inhibit" refers to decreasing the cellular function of a PPAR. The PPAR function may be the interaction with a natural binding partner or catalytic activity.

The term "cell phenotype" refers to the outward appearance of a cell or tissue or the function of the cell or tissue. Examples of cell or tissue phenotype are cell size (reduction or enlargement), cell proliferation (increased or decreased numbers of cells), cell differentiation (a change or absence of a change in cell shape), cell survival, apoptosis (cell death), or the utilization of a metabolic nutrient (e.g., glucose uptake). Changes or the absence of changes in cell phenotype are readily measured by techniques known in the art.

The term "cell proliferation" refers to the rate at which a group of cells divides. The number of cells growing in a vessel can be quantified by a person skilled in the art when that person visually counts the number of cells in a defined area using a common light microscope. Alternatively, cell proliferation rates can be quantified by laboratory apparatus that optically measure the density of cells in an appropriate medium.

The term "contacting" as used herein refers to bringing a compound of this invention and a target PPAR together in such a manner that the compound can affect the activity of the PPAR, either directly; i.e., by interacting with the PPAR itself, or indirectly; i.e., by interacting with another molecule on which the activity of the PPAR is dependent. Such "contacting" can be accomplished in a test tube, a petri dish, a test organism (e.g., murine, hamster or primate), or the like. In a test tube, contacting may involve only a compound and a PPAR of interest or it may involve whole cells. Cells may also be maintained or grown in cell culture dishes and contacted with a compound in that environment. In this context, the ability of a particular compound to affect a PPAR related disorder; i.e., the IC₅₀ of the compound can be determined before use of the compounds in vivo with more complex living organisms is attempted. For cells outside the organism, multiple methods exist, and are well-known to those skilled in the art, to get the PPARs in contact with the compounds including, but not limited to, direct cell microinjection and numerous transmembrane carrier techniques.

The term "modulate" refers to the ability of a compound of the invention to alter the function of a PPAR. A modulator may activate the activity of a PPAR, may activate or inhibit the activity of a PPAR depending on the concentration of the compound exposed to the PPAR, or may inhibit the activity of a PPAR. The term "modulate" also refers to altering the function of a PPAR by increasing or decreasing the probability that a complex forms between a PPAR and a natural binding partner. A modulator may increase the probability that such a complex forms between the PPAR and the natural binding partner, may increase or decrease the probability that a complex forms between the PPAR and the natural binding partner depending on the concentration of the compound exposed to the PPAR, and or may decrease the probability that a complex forms between the PPAR and the natural binding partner.

The term "monitoring" refers to observing the effect of adding the compound of the invention to the cells of the method. The effect can be manifested in a change in cell phenotype, cell proliferation, PPAR activity, or in the interaction between a PPAR and a natural binding partner. Of course, the term "monitoring" includes detecting whether a change has in fact occurred or not.

### A. Exemplary Assays

The following assay methods are provided by way of example only. Compounds may be tested for their ability to bind to bPPAR-gamma, hPPAR-alpha, or PPAR-delta using a Scintillation Proximity Assay (SPA). The PPAR ligand binding domain (LBO) may be expressed in E. coli as polyHis tagged fusion proteins and purified. The LBO is then labeled with biotin and immobilized on streptavidin modified scintillation proximity beads. The beads are then incubated with a constant amount of the appropriate radioligand eH-BRL 49653 for PPARγ, 2-(4(2-(2,3-Ditritio-1-heptyl-3-(2,4-difluorophenyl)ureido) ethyl)phenoxy)-2 methyl butanoic acid (described in WO1008002) for hPPAR-alpha and GW 2433 (see Brown, P. J et al . *Chem. Biol.* **1997**, 4, 909-918. For the structure and synthesis of this ligand) for PPAR-delta) and variable concentrations of test compound, and after equilibration the radioactivity bound to the beads is measured by a scintillation counter. The amount of nonspecific binding, as assessed by control wells containing 50 µM of the corresponding unlabelled ligand, is subtracted from each data point. For each compound tested, plots of ligand concentration vs. CPM of radioligand bound are constructed and apparent K, values are estimated from nonlinear least squares fit of the data assuming simple competitive binding. The details of this assay have been reported elsewhere (see, Blanchard, S. G. et. al., "Development of a Scintillation Proximity Assay for Peroxisome Proliferator-Activated Receptor gamma Ligand Binding Domain" Anal. Biochem. 1998,257,112-119).

### B. Tranfection Assays

The following transfection assay methods are provided by way of example only. Compounds may be screened for functional potency in transient transfection assays in CV-1 cells for their ability to activate the PPAR subtypes (transactivation assay). A previously established chimeric receptor system was utilized to allow comparison of the relative transcriptional activity of the receptor subtypes on the same target gene and to prevent endogenous receptor activation from complicating the interpretation of results. See, for example, Lehmann, J. M.; Moore, L. B.; Smith-Oliver, T. A; Wilkinson, W.O.; Willson, T. M.; Kliewer, S. A., An antidiabetic thiazolidinedione is a high affinity ligand for peroxisome proliferator-activated receptor γ (PPARγ), *J*. *Biol. Chem.,* 1995, 270, 12953-6. The ligand binding domains for murine and human PPAR-alpha, PPAR-gamma, and PPAR-delta are each fused to the yeast transcription factor GAL4 DNA binding domain. CV-1 cells were transiently transfected with expression vectors for the respective PPAR chimera along with a reporter construct containing five copies of the GAL4 DNA binding site driving expression of secreted placental alkaline phosphatase (SPAP) and p-galactosidase. After 16 h, the medium is exchanged to DME medium supplemented with 10% delipidated fetal calf serum and the test compound at the appropriate concentration. After an additional 24 h, cell extracts are prepared and assayed for alkaline phosphatase and pgalactosidase activity. Alkaline phosphatase activity was corrected for transfection efficiency using the p-galactosidase activity as an internal standard (see, for example, Kliewer, S. A., et. al. Cell 1995, 83, 813-819. Rosiglitazone is used as a positive control in the hPPARγ assay. The positive control in the hPPAR-alpha and hPPAR-delta assays was 2-[4-(2-(3-(4-fluorophenyl)-lheptylureido)ethyl)-phenoxy]-2-methylpropionic acid, which can be prepared as described in Brown, Peter J., et. al. Synthesis (7), 778-782 (1997), or patent publication WO 9736579.

### III. TARGET DISEASES TO BE TREATED

In another aspect, the present invention relates to a method of treating a disease comprising identifying a patient in need thereof, and administering a therapeutically effective amount of a compound of Formula I, as described herein, to the patient.

Biological processes modulated by PPAR are those modulated by receptors, or receptor combinations, which are responsive to the PPAR receptor ligands described herein. These processes includem for example, plasma lipid transport and fatty acid catabolism, regulation of insulin sensitivity and blood glucose levels, which are involved in hypoglycemia/hyperinsulinemia (resulting from, for example, abnormal pancreatic beta cell function, insulin secreting tumors and/or autoimmune hypoglycemia due to autoantibodies to insulin, the insulin receptor, or autoantibodies that are stimulatory to pancreatic beta cells), macrophage differentiation which lead to the formation of atherosclerotic plaques, inflammatory response, carcinogenesis, hyperplasia, and adipocyte differentiation.

Non-insulin-dependent diabetes mellitus (NIDDM), or Type 2 diabetes, is the more common form of diabetes, with 90-95% of hyperglycemic patients experiencing this form of the disease. Resistance to the metabolic actions of insulin is one of the key features of non-insulin dependent diabetes (NIDDM). Insulin resistance is characterized by impaired uptake and utilization of glucose in insulin-sensitive target organs, for example, adipocytes and skeletal muscle, and by impaired inhibition of hepatic glucose output. The functional insulin deficiency and the failure of insulin to suppress hepatic glucose output results in fasting hyperglycemia. Pancreatic β-cells compensate for the insulin resistance by secreting increased levels of insulin. However, the β-cells are unable to maintain this high output of insulin, and, eventually, the glucose-induced insulin secretion falls, leading to the deterioration of glucose homeostasis and to the subsequent development of overt diabetes.

Compelling evidence has shown that PPARγ is a valuable molecular target for development of drugs for treatment of insulin resistance (see Willson, et al. J. Med. Chem. 43: 527-550 (2000)). In fact, PPARγ agonists rosiglitazone (Avandia) and pioglitazone (Actos) are insulin sensitizers and are currently marketed drugs for treatment of type 2 diabetes.

Obesity is an excessive accumulation of adipose tissue. Recent work in this area indicates that PPARγ plays a central role in the adipocyte gene expression and differentiation. Excess adipose tissue is associated with the development of serious medical conditions, for example, non-insulin-dependent diabetes mellitus (NIDDM), hypertension, coronary artery disease, hyperlipidemia obesity and certain malignancies. The adipocyte may also influence glucose homeostasis through the production of tumor necrosis factor α (TNFα) and other molecules. PPARγ activators, in particular Troglitazone®, have been found to convert cancerous tissue to normal cells in liposarcoma, a tumor of fat (PNAS 96:3951-3956, 1999). Therefore, PPARγ activators may be useful in the treatment of obesity and breast and colon cancer.

Moreover, PPARγ activators, for example Troglitazone®, have been implicated in the treatment of polycystic ovary syndrome (PCO). This is a syndrome in women that is characterized by chronic anovulation and hyperandrogenism. Women with this syndrome often have insulin resistance and an increased risk for the development of non insulin-dependent diabetes mellitus. (Dunaif, Scott, Finegood, Quintana, Whitcomb, J. Clin. Endocrinol. Metab., 81:3299,1996.

Furthermore, PPARγ activators have recently been discovered to increase the production of progesterone and inhibit steroidogenesis in granulosa cell cultures and therefore may be useful in the treatment of climacteric. (USP 5,814,647 Urban et al. September 29,1998; B. Lohrke et al. Journal of Edocrinology, 159,429-39, 1998). Climacteric is defined as the syndrome of endocrine, somatic and psychological changes occurring at the termination of the reproductive period in the female.

PPARα is activated by a number of medium and long-chain fatty acids and is involved in stimulating β-oxidation of fatty acids in tissues such as liver, heart, skeletal muscle, and brown adipose tissue (Isseman and Green, supra; Beck et al., Proc. R. Soc. Lond. 247:83-87,1992; Gottlicher et al., Proc. Natl. Acad. Sci. USA 89:4653-4657, 1992). Pharmacological PPARα activators, for example fenofibrate, clofibrate, genfibrozil, and bezafibratem are also involved in substantial reduction in plasma triglycerides along with moderate reduction in LDL cholesterol, and they are used particularly for the treatment of hypertriglyceridemia, hyperlipidemia and obesity. PPARα is also known to be involved in inflammatory disorders. (Schoonjans, K., Current Opinion in Lipidology, 8, 159-66, 1997).

PPARα agonists may also be useful in raising HDL levels and therefore may be useful in treating atherosclerotic diseases. (Leibowitz et al.; WO/9728149). Atherosclerotic diseases include vascular disease, coronary heart disease, cerebrovascular disease and peripheral vessel disease. Coronary heart disease includes CHD death, myocardial infarction, and coronary revascularization. Cerebrovascular disease includes ischemic or hemorrhagic stroke and transient ischemic attacks.

The third subtype ofPPARs, PPARδ (PPARβ, NUC1), is broadly expressed in the body and has been shown to be a valuable molecular target for treatment of dyslipedimia and other diseases. For example, in a recent study in insulin-resistant obese rhesus monkeys, a potent and selective PPARδ compound was shown to decrease VLDL and increase HDL in a dose response manner (Oliver et al., Proc. Natl. Acad. Sci. U. S. A.98: 5305, 2001).

Compounds described herein may be activating both PPARα and PPARγ, or PPARδ and PPARγ, or all three PPAR subtypes and therefore may be used in the treatment of dyslipidemia associated with atherosclerosis, non-insulin dependent diabetes mellitus, metabolic syndrome X, (Staels, B. et al., Curr. Pharm. Des., 3 (1),1-14 (1997)) and familial combined hyperlipidemia (FCH). Metabolic syndrome X is the syndrome characterized by an initial insulin resistant state, generating hyperinsulinaemia, dyslipidaemia and impaired glucose tolerance, which can progress to non-insulin dependent diabetes mellitus (Type 2 diabetes), characterized by hyperglycemia. FCH is characterized by hypercholesterolemia and hypertriglyceridemia within the same patient and family.

Thus, in certain embodiments, the disease to be treated by the methods of the present invention is selected from the group consisting of obesity, diabetes, hyperinsulinemia, metabolic syndrome X, polycystic ovary syndrome, climacteric, disorders associated with oxidative stress, inflammatory response to tissue injury, pathogenesis of emphysema, ischemia-associated organ injury, doxorubicin-induced cardiac injury, drug-induced hepatotoxicity, atherosclerosis, and hypertoxic lung injury.

### IV. PHARMACEUTICAL COMPOSITIONS

In another aspect, the present invention relates to a pharmaceutical composition comprising a compound of Formula I, as described herein, and a pharmaceutically acceptable diluent, excipient, or carrier.

The term "pharmaceutical composition" refers to a mixture of a compound of the invention with other chemical components, such as carriers, diluents or excipients. The pharmaceutical composition facilitates administration of the compound to an organism. Multiple techniques of administering a compound exist in the art including, but not limited to: intravenous, oral, aerosol, parenteral, ophthalmic, pulmonary and topical administration. Pharmaceutical compositions can also be obtained by reacting compounds with inorganic or organic acids such as hydrochloric acid, hydrobromic acid, sulfuric acid, nitric acid, phosphoric acid, methanesulfonic acid, ethanesulfonic acid, p-toluenesulfonic acid, salicylic acid and the like.

The term "carrier" refers to relatively nontoxic chemical compounds or agents. Such carriers may facilitate the incorporation of a compound into cells or tissues. For example, human serum albumin (HSA) is a commonly utilized carrier as it facilitates the uptake of many organic compounds into the cells or tissues of an organism.

The term "diluent" refers to chemical compounds that are used to dilute the compound of interest prior to delivery. Diluents can also be used to stabilize compounds because they can provide a more stable environment. Salts dissolved in buffered solutions (providing pH control) are utilized as diluents in the art. One commonly used buffered solution is phosphate buffered saline. It is a buffer found naturally in the blood system. Since buffer salts can control the pH of a solution at low concentrations, a buffered diluent rarely modifies the biological activity of a compound.

The term "physiologically acceptable" refers to a carrier or diluent that does not abrogate the biological activity or properties of the compound, and is nontoxic.

The term "pharmaceutically acceptable salt" refers to a formulation of a compound that does not cause significant irritation to an organism to which it is administered and does not abrogate the biological activity and properties of the compound. Pharmaceutically acceptable salts may be obtained by reacting a compound of the invention with acids such as hydrochloric acid, hydrobromic acid, sulfuric acid, nitric acid, phosphoric acid, methanesulfonic acid, ethanesulfonic acid, p-toluenesulfonic acid, salicylic acid and the like. Pharmaceutically acceptable salts may also be obtained by reacting a compound of the invention with a base to form a salt such as an ammonium salt, an alkali metal salt, such as a sodium or a potassium salt, an alkaline earth metal salt, such as a calcium or a magnesium salt, a salt of organic bases such as dicyclohexylamine, N-methyl-D-glucamine, tris(hydroxymethyl)methylamine, and salts with amino acids such as arginine, lysine, and the like, or by other methods known in the art.

A "prodrug" refers to an agent that is converted into the parent drug *in vivo.* Prodrugs are often useful because, in some situations, they may be easier to administer than the parent drug. They may, for instance, be bioavailable by oral administration whereas the parent is not. The prodrug may also have improved solubility in pharmaceutical compositions over the parent drug. An example, without limitation, of a prodrug would be a compound of the present invention which is administered as an ester (the "prodrug") to facilitate transmittal across a cell membrane where water solubility is detrimental to mobility but which then is metabolically hydrolyzed to the carboxylic acid, the active entity, once inside the cell where water-solubility is beneficial. A further example of a prodrug might be a short peptide (polyaminoacid) bonded to an acid group where the peptide is metabolized to reveal the active moiety.

The compounds described herein can be administered to a human patient *per se,* or in pharmaceutical compositions where they are mixed with other active ingredients, as in combination therapy, or suitable carriers or excipient(s). Techniques for formulation and administration of the compounds of the instant application may be found in "Remington's Pharmaceutical Sciences," 20th ed. Edited by Alfonso Gennaro, 2000.

### A. Routes Of Administration

Suitable routes of administration may, for example, include oral, rectal, transmucosal, pulmonary, ophthalmic or intestinal administration; parenteral delivery, including intramuscular, subcutaneous, intravenous, intramedullary injections, as well as intrathecal, direct intraventricular, intraperitoneal, intranasal, or intraocular injections.

Alternately, one may administer the compound in a local rather than systemic manner, for example, via injection of the compound directly into an organ, often in a depot or sustained release formulation. Furthermore, one may administer the drug in a targeted drug delivery system, for example, in a liposome coated with organ-specific antibody. The liposomes will be targeted to and taken up selectively by the organ.

### B. Composition/Formulation

The pharmaceutical compositions of the present invention may be manufactured in a manner that is itself known, e.g., by means of conventional mixing, dissolving, granulating, dragee-making, levigating, emulsifying, encapsulating, entrapping or compression processes.

Pharmaceutical compositions for use in accordance with the present invention thus may be formulated in conventional manner using one or more physiologically acceptable carriers comprising excipients and auxiliaries which facilitate processing of the active compounds into preparations which can be used pharmaceutically. Proper formulation is dependent upon the route of administration chosen. Any of the well-known techniques, carriers, and excipients may be used as suitable and as understood in the art; *e.g.*, in Remington's Pharmaceutical Sciences, above.

For intravenous injections, the agents of the invention may be formulated in aqueous solutions, preferably in physiologically compatible buffers such as Hanks's solution, Ringer's solution, or physiological saline buffer. For transmucosal administration, penetrants appropriate to the barrier to be permeated are used in the formulation. Such penetrants are generally known in the art. For other parenteral injections, the agents of the invention may be formulated in aqueous or nonaqueous solutions, preferably with physiologically compatible buffers or excipients. Such excipients are generally known in the art.

For oral administration, the compounds can be formulated readily by combining the active compounds with pharmaceutically acceptable carriers or excipients well known in the art. Such carriers enable the compounds of the invention to be formulated as tablets, powders, pills, dragees, capsules, liquids, gels, syrups, elixirs, slurries, suspensions and the like, for oral ingestion by a patient to be treated. Pharmaceutical preparations for oral use can be obtained by mixing one or more solid excipient with one or more compound of the invention, optionally grinding the resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as: for example, maize starch, wheat starch, rice starch, potato starch, gelatin, gum tragacanth, methylcellulose, microcrystalline cellulose, hydroxypropylmethylcellulose, sodium carboxymethylcellulose; or others such as: polyvinylpyrrolidone (PVP or povidone) or calcium phosphate. If desired, disintegrating agents may be added, such as the cross-linked croscarmellose sodium, polyvinyl pyrrolidone, agar, or alginic acid or a salt thereof such as sodium alginate.

Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Pharmaceutical preparations which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. All formulations for oral administration should be in dosages suitable for such administration.

For buccal or sublingual administration, the compositions may take the form of tablets, lozenges, or gels formulated in conventional manner.

For administration by inhalation, the compounds for use according to the present invention are conveniently delivered in the form of an aerosol spray presentation from pressurized packs or a nebuliser, with the use of a suitable propellant, *e.g*., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of, *e.g.*, gelatin for use in an inhaler or insufflator may be formulated containing a powder mix of the compound and a suitable powder base such as lactose or starch.

The compounds may be formulated for parenteral administration by injection, e.g., by bolus injection or continuous infusion. Formulations for injection may be presented in unit dosage form, *e.g.*, in ampoules or in multi-dose containers, with an added preservative. The compositions may take such forms as suspensions, solutions or emulsions in oily or aqueous vehicles, and may contain formulatory agents such as suspending, stabilizing and/or dispersing agents.

Pharmaceutical formulations for parenteral administration include aqueous solutions of the active compounds in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

Alternatively, the active ingredient may be in powder form for constitution with a suitable vehicle, *e.g.,* sterile pyrogen-free water, before use.

The compounds may also be formulated in rectal compositions such as suppositories or retention enemas, *e.g*., containing conventional suppository bases such as cocoa butter or other glycerides.

In addition to the formulations described previously, the compounds may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

A pharmaceutical carrier for the hydrophobic compounds of the invention is a cosolvent system comprising benzyl alcohol, a nonpolar surfactant, a water-miscible organic polymer, and an aqueous phase. The cosolvent system may be a 10% ethanol, 10% polyethylene glycol 300, 10% polyethylene glycol 40 castor oil (PEG-40 castor oil) with 70% aqueous solution. This cosolvent system dissolves hydrophobic compounds well, and itself produces low toxicity upon systemic administration. Naturally, the proportions of a cosolvent system may be varied considerably without destroying its solubility and toxicity characteristics. Furthermore, the identity of the cosolvent components may be varied: for example, other low-toxicity nonpolar surfactants may be used instead of PEG-40 castor oil, the traction size of polyethylene glycol 300 may be varied; other biocompatible polymers may replace polyethylene glycol, *e.g.,* polyvinyl pyrrolidone; and other sugars or polysaccharides maybe included in the aqueous solution.

Alternatively, other delivery systems for hydrophobic pharmaceutical compounds may be employed. Liposomes and emulsions are well known examples of delivery vehicles or carriers for hydrophobic drugs. Certain organic solvents such as N-methylpyrrolidone also may be employed, although usually at the cost of greater toxicity. Additionally, the compounds may be delivered using a sustained-release system, such as semipermeable matrices of solid hydrophobic polymers containing the therapeutic agent. Various sustained-release materials have been established and are well known by those skilled in the art. Sustained-release capsules may, depending on their chemical nature, release the compounds for a few weeks up to over 100 days. Depending on the chemical nature and the biological stability of the therapeutic reagent, additional strategies for protein stabilization may be employed.

Many of the compounds of the invention may be provided as salts with pharmaceutically compatible counterions. Pharmaceutically compatible salts may be formed with many acids, including but not limited to hydrochloric, sulfuric, acetic, lactic, tartaric, malic, succinic, etc. Salts tend to be more soluble in aqueous or other protonic solvents than are the corresponding free acid or base forms.

### V. TREATMENT METHODS, DOSAGES AND COMBINATION THERAPIES

The term "patient" means all mammals including humans. Examples of patients include humans, cows, dogs, cats, goats, sheep, pigs, and rabbits.

The term "therapeutically effective amount" as used herein refers to that amount of the compound being administered which will relieve to some extent one or more of the symptoms of the disease, condition or disorder being treated. In reference to the treatment of diabetes or dyslipidemia a therapeutically effective amount refers to that amount which has the effect of (1) reducing the blood glucose levels; (2) normalizing lipids, e.g. triglycerides, low-density lipoprotein; and/or (3) relieving to some extent (or, preferably, eliminating) one or more symptoms associated with the disease, condition or disorder to be treated.

The compositions containing the compound(s) described herein can be administered for prophylactic and/or therapeutic treatments. In therapeutic applications, the compositions are administered to a patient already suffering from a disease, condition or disorder mediated, modulated or involving the PPARs, including but not limited to metabolic diseases, conditions, or disorders, as described above, in an amount sufficient to cure or at least partially arrest the symptoms of the disease, disorder or condition. Amounts effective for this use will depend on the severity and course of the disease, disorder or condition, previous therapy, the patient's health status and response to the drugs, and the judgment of the treating physician. It is considered well within the skill of the art for one to determine such therapeutically effective amounts by routine experimentation (e.g., a dose escalation clinical trial).

In prophylactic applications, compositions containing the compounds described herein are administered to a patient susceptible to or otherwise at risk of a particular disease, disorder or condition mediated, modulated or involving the PPARs, including but not limited to metabolic diseases, conditions, or disorders, as described above. Such an amount is defined to be a "prophylactically effective amount or dose." In this use, the precise amounts also depend on the patient's state of health, weight, and the like. It is considered well within the skill of the art for one to determine such prophylactically effective amounts by routine experimentation (e.g., a dose escalation clinical trial).

The terms "enhance" or "enhancing" means to increase or prolong either in potency or duration a desired effect. Thus, in regard to enhancing the effect of therapeutic agents, the term "enhancing" refers to the ability to increase or prolong, either in potency or duration, the effect of other therapeutic agents on a system. An "enhancing-effective amount," as used herein, refers to an amount adequate to enhance the effect of another therapeutic agent in a desired system. When used in a patient, amounts effective for this use will depend on the severity and course of the disease, disorder or condition (including, but not limited to, metabolic disorders), previous therapy, the patient's health status and response to the drugs, and the judgment of the treating physician. It is considered well within the skill of the art for one to determine such enhancing-effective amounts by routine experimentation.

Once improvement of the patient's conditions has occurred, a maintenance dose is administered if necessary. Subsequently, the dosage or the frequency of administration, or both, can be reduced, as a function of the symptoms, to a level at which the improved disease, disorder or condition is retained. When the symptoms have been alleviated to the desired level, treatment can cease. Patients can, however, require intermittent treatment on a long-term basis upon any recurrence of symptoms.

The amount of a given agent that will correspond to such an amount will vary depending upon factors such as the particular compound, disease condition and its severity, the identity (e.g., weight) of the subject or host in need of treatment, but can nevertheless be routinely determined in a manner known in the art according to the particular circumstances surrounding the case, including, e.g., the specific agent being administered, the route of administration, the condition being treated, and the subject or host being treated. In general, however, doses employed for adult human treatment will typically be in the range of 0.02-5000 mg per day, preferably 1-1500 mg per day. The desired dose may conveniently be presented in a single dose or as divided doses administered at appropriate intervals, for example as two, three, four or more sub-doses per day.

In certain instances, it may be appropriate to administer at least one of the compounds described herein (or a pharmaceutically acceptable salt, ester, amide, prodrug, or solvate) in combination with another therapeutic agent. By way of example only, if one of the side effects experienced by a patient upon receiving one of the compounds herein is hypertension, then it may be appropriate to administer an anti-hypertensive agent in combination with the initial therapeutic agent. Or, by way of example only, the therapeutic effectiveness of one of the compounds described herein may be enhanced by administration of an adjuvant (i.e., by itself the adjuvant may only have minimal therapeutic benefit, but in combination with another therapeutic agent, the overall therapeutic benefit to the patient is enhanced). Or, by way of example only, the benefit of experienced by a patient may be increased by administering one of the compounds described herein with another therapeutic agent (which also includes a therapeutic regimen) that also has therapeutic benefit. By way of example only, in a treatment for diabetes involving adiministration of one of the compounds described herein, increased therapeutic benefit may result by also providing the patient with another therapeutic agent for diabetes. In any case, regardless of the disease, disorder or condition being treated, the overall benefit experienced by the patient may simply be additive of the two therapeutic agents or the patient may experience a synergistic benefit.

Specific, non-limiting examples of possible combination therapies include use of the compound of formula (I) with: (a) stating and/or other lipid lowering drugs for example MTP inhibitors and LDLR upregulators; (b) antidiabetic agents, e.g. metformin, sulfonylureas, or PPAR-gamma, PPAR-alpha and PPAR-alpha/gamma modulators (for example thiazolidinediones such as e.g. Pioglitazone and Rosiglitazone); and (c) antihypertensive agents such as angiotensin antagonists, e.g., telmisartan, calcium channel antagonists, e.g. lacidipine and ACE inhibitors, e.g., enalapril.

In any case, the multiple therapeutic agents (one of which is one of the compounds described herein) may be administered in any order or even simultaneously. If simultaneously, the multiple therapeutic agents may be provided in a single, unified form, or in multiple forms (by way of example only, either as a single pill or as two separate pills). One of the therapeutic agents may be given in multiple doses, or both may be given as multiple doses. If not simultaneous, the timing between the multiple doses may vary from more than zero weeks to less than four weeks.

### VI. SYNTHESIS OF THE COMPOUNDS OF THE INVENTION

Compounds of the present invention may be synthesized using standard synthetic techniques known to those of skill in the art or using methods known in the art in combination with methods described herein. As a guide the following synthetic methods may be utilized.

### A. Formation of Covalent Linkages by Reaction of an Electrophile with a Nucleophile

Selected examples of covalent linkages and precursor functional groups which yield them are given in the Table entitled "Examples of Covalent Linkages and Precursors Thereof." Precursor functional groups are shown as electrophilic groups and nucleophilic groups. The functional group on the organic substance may be attached directly, or attached via any useful spacer or linker as defined below.

**Table 1**

| Examples of Covalent Linkages and Precursors Thereof | | |
|---|---|---|
| Covalent Linkage Product. | Electrophile | Nucleophile |
| Carboxamides | Activated esters | amines/anilines |
| Carboxamides | acyl azides | amines/anilines |
| Carboxamides | acyl halides | amines/anilines |
| Esters | acyl halides | alcohols/phenols |
| Esters | acyl nitriles | alcohols/phenols |
| Carboxamides | acyl nitriles | amines/anilines |
| Imines | Aldehydes | amines/anilines |
| Hydrazones | aldehydes or ketones | Hydrazines |
| Oximes | aldehydes or ketones | Hydroxylamines |
| Alkyl amines | alkyl halides | amines/anilines |
| Esters | alkyl halides | carboxylic acids |
| Thioethers | alkyl halides | Thiols |
| Ethers | alkyl halides | alcohols/phenols |
| Thioethers | alkyl sulfonates | Thiols |
| Esters | alkyl sulfonates | carboxylic acids |
| Ethers | alkyl sulfonates | alcohols/phenols |
| Esters | Anhydrides | alcohols/phenols |
| Carboxamides | Anhydrides | amines/anilines |
| Thiophenols | aryl halides | Thiols |
| Aryl amines | aryl halides | Amines |
| Thioethers | Azindines | Thiols |
| Boronate esters | Boronates | Glycols |
| Carboxamides | carboxylic acids | amines/anilines |
| Esters | carboxylic acids | Alcohols |
| hydrazines | Hydrazides | carboxylic acids |
| N-acylureas or Anhydrides | carbodiimides | carboxylic acids |
| Esters | diazoalkanes | carboxylic acids |
| Thioethers | Epoxides | Thiols |
| Thioethers | haloacetamides | Thiols |
| Ammotriazines | halotriazines | amines/anilines |
| Triazinyl ethers | halotriazines | alcohols/phenols |
| Amidines | imido esters | amines/anilines |
| Ureas | Isocyanates | amines/anilines |
| Urethanes | Isocyanates | alcohols/phenols |
| Thioureas | isothiocyanates | amines/anilines |
| Thioethers | Maleimides | Thiols |
| Phosphite esters | phosphoramidites | Alcohols |
| Silyl ethers | silyl halides | Alcohols |
| Alkyl amines | sulfonate esters | amines/anilines |
| Thioethers | sulfonate esters | Thiols |
| Esters | sulfonate esters | carboxylic acids |
| Ethers | sulfonate esters | Alcohols |
| Sulfonamides | sulfonyl halides | amines/anilines |
| Sulfonate esters | sulfonyl halides | phenols/alcohols |

In general, carbon electrophiles are susceptible to attack by complementary nucleophiles, including carbon nucleophiles, wherein an attacking nucleophile brings an electron pair to the carbon electrophile in order to form a new bond between the nucleophile and the carbon electrophile.

Suitable carbon nucleophiles include, but are not limited to alkyl, alkenyl, aryl and alkynyl Grignard, organolithium, organozinc, alkyl-, alkenyl , aryl- and alkynyl-tin reagents (organostannanes), alkyl-, alkenyl-, aryl- and alkynyl-borane reagents (organoboranes and organoboronates); these carbon nucleophiles have the advantage of being kinetically stable in water or polar organic solvents. Other carbon nucleophiles include phosphorus ylids, enol and enolate reagents; these carbon nucleophiles have the advantage of being relatively easy to generate from precursors well known to those skilled in the art of synthetic organic chemistry. Carbon nucleophiles, when used in conjunction with carbon electrophiles, engender new carbon-carbon bonds between the carbon nucleophile and carbon electrophile.

Non-carbon nucleophiles suitable for coupling to carbon electrophiles include but are not limited to primary and secondary amines, thiols, thiolates, and thioethers, alcohols, alkoxides, azides, semicarbazides, and the like. These non-carbon nucleophiles, when used in conjunction with carbon electrophiles, typically generate heteroatom linkages (C-X-C), wherein X is a hetereoatom, e. g, oxygen or nitrogen.

### B. Use of Protecting Groups

The term "protecting group" refers to chemical moieties that block some or all reactive moieties and prevent such groups from participating in chemical reactions until the protective group is removed. It is preferred that each protective group be removable by a different means. Protective groups that are cleaved under totally disparate reaction conditions fulfill the requirement of differential removal. Protective groups can be removed by acid, base, and hydrogenolysis. Groups such as trityl, dimethoxytrityl, acetal and t-butyldimethylsilyl are acid labile and may be used to protect carboxy and hydroxy reactive moieties in the presence of amino groups protected with Cbz groups, which are removable by hydrogenolysis, and Fmoc groups, which are base labile. Carboxylic acid and hydroxy reactive moieties may be blocked with base labile groups such as, without limitation, methyl, ethyl, and acetyl in the presence of amines blocked with acid labile groups such as t-butyl carbamate or with carbamates that are both acid and base stable but hydrolytically removable.

Carboxylic acid and hydroxy reactive moieties may also be blocked with hydrolytically removable protective groups such as the benzyl group, while amine groups capable of hydrogen bonding with acids may be blocked with base labile groups such as Fmoc. Carboxylic acid reactive moieties may be blocked with oxidatively-removable protective groups such as 2,4-dimethoxybenzyl, while co-existing amino groups may be blocked with fluoride labile silyl carbamates.

Allyl blocking groups are useful in then presence of acid- and base- protecting groups since the former are stable and can be subsequently removed by metal or pi-acid catalysts. For example, an allyl-blocked carboxylic acid can be deprotected with a Pd₀₋catalyzed reaction in the presence of acid labile t-butyl carbamate or base-labile acetate amine protecting groups. Yet another form of protecting group is a resin to which a compound or intermediate may be attached. As long as the residue is attached to the resin, that functional group is blocked and cannot react. Once released from the resin, the functional group is available to react.

Typically blocking/protecting groups may be selected from:

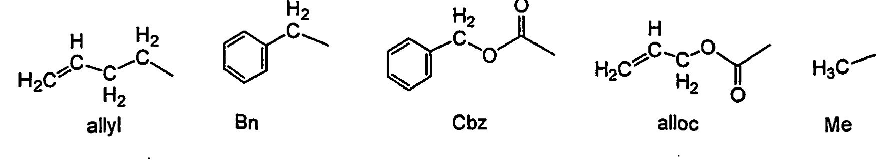

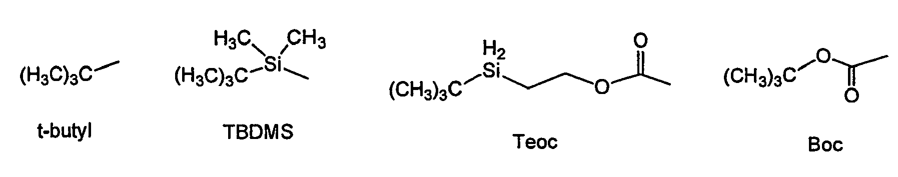

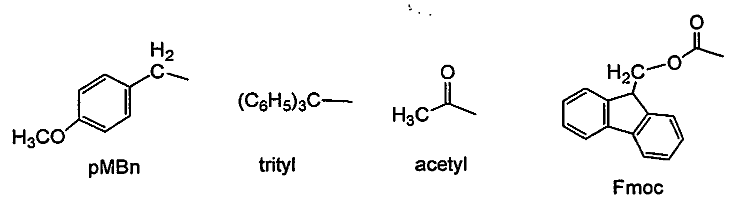

Other protecting groups are described in Greene and Wuts, Protective Groups in Organic Synthesis, 3rd Ed., John Wiley & Sons, New York, NY, 1999, which is incorporated herein by reference in its entirety.

### C. Synthetic Schemes

Compounds of the present invention may be synthesized using the general synthetic procedures and examples set forth below.

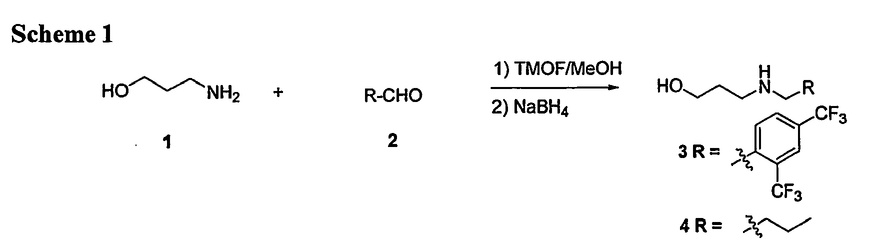

### EXAMPLE 1A: Synthesis of 3-(2,4-Bis-trifluoromethyl-benzylamino)-propan-1-ol (3) (Scheme 1).

3-Hydroxypropylamine (5.62 mL, 73.5 mmol, 1.2 equiv.) was dissolved in 250 mL of TMOF/MeOH (1:5) (TMOF = trimethyl orthoformate) and then 2,4-bis(trifluoromethyl)benzaldehyde (14.83g, 61.2 mmol, 1.0 equiv.) was added to this solution at room temperature with stirring. The resulting solution was stirred at rt for 6 hours and then cooled to 0°C. NaBH₄ was added to the cooled reaction solution in portions with vigorous stirring. After TLC indicated the reduction complete, the reaction mixture was concentrated under reduced pressure. The residue was diluted with 250 mL of ethyl acetate and washed with water, brine and then dried over Na₂SO₄. After removal of solvent, 17.1 g (93% yield) colorless oil was obtained as desired 3-(2,4-Bis-trifluoromethyl-benzylamino)-propan-1-ol (3). ¹H NMR (400 MHz, CDCl₃), δ (ppm): 7.9 (s, 1H), 7.75 (m, 2H), 4.0 (s, 2H), 3.8 (t, 2H), 2.85 (t, 2H), 1.76 (m, 2H).

### EXAMPLE 1B: Synthesis of 3-Butylamino-propan-1-ol (4) (Scheme 1).

Compound (4) was synthesized following the procedure described for compound (3). ¹H NMR (400 MHz, CDCl₃), δ (ppm): 3.82 (t, 2H), 2.90 (br, 2H), 2.88 (t, 2H), 2.61 (t, 2H), 1.68 (m, 2H), 1.45 (m, 2H), 1.35 (m, 2H), 0.90 (t, 3H).

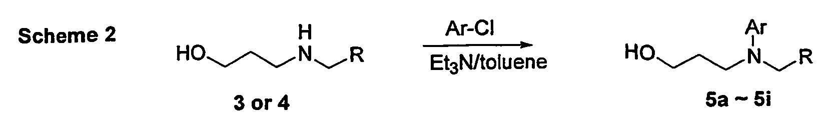

| | **R** | **Ar** |
|---|---|---|
| **5a** | 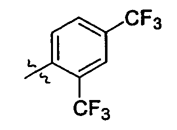 | 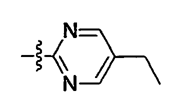 |
| **5b** | 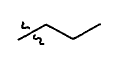 | 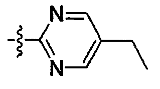 |
| **5c** | 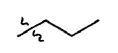 | 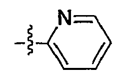 |
| **5d** | 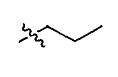 | 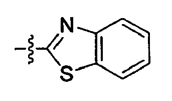 |
| **5e** | 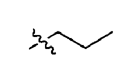 | 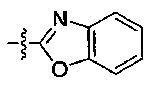 |
| **5f** | 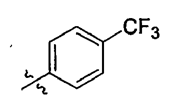 | 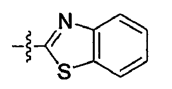 |
| **5g** | 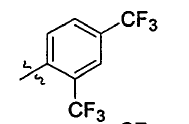 | 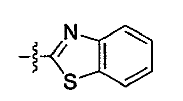 |
| **5h** | 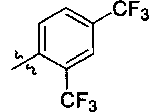 | 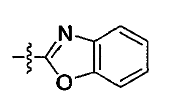 |
| **5i** | 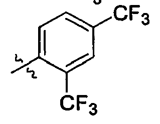 | 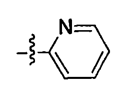 |

### EXAMPLE 2A: Synthesis of 3-[(2,4-Bis-trifluoromethyl-benzyl)-(5-ethyl-pyrimidin-2-yl)-amino]-propan-1-ol (5a) (Scheme 2).

To a high pressure flask was added intermediate (3) (12.23g, 40.6 mmol, 1.0 equiv.), 2-chloro-5-ethylpyrimidine (4.9 mL, 40.6 mmol, 1.0 equiv.), triethylamine (11.3 mL, 81.2 mmol, 2.0 equiv.) and 50 mL of toluene. After the flask was sealed, it was heated to 180°C with stirring. After reaction at same temperature for 48 hours, the reaction mixture was cooled to room temperature and then diluted with 100 mL of ethyl acetate. The resulting solution was washed with water, brine and the dried over Na₂SO₄. After removal of solvent, the residue was purified by chromatography to give 7.7 g (46% yield) of product (5a) as bright brown solid. ¹H NMR (400 MHz, CDCl₃), δ (ppm): 8.15 (s, 2H), 7.90 (s, 1H), 7.67 (d, 1H), 7.30 (d, 1H), 5.02 (s, 2H), 3.71 (m, 2H), 3.53 (m, 2H), 2.42 (q, 2H), 1.75 (m, 2H), 1.15 (t, 3H).

### EXAMPLE 2B: Compounds 5b -5i were prepared followed the procedure described for 5a (Scheme 2).

3-[Butyl-(5-ethyl-pyrimidin-2-yl)-amino]-propan-1-ol (5b). ¹H NMR (400 MHz, CDCl₃), δ (ppm): 8.10 (s, 2H), 5.15 (s, 1H), 3.71 (t, 3H), 3.45 (m, 4H), 2.42 (q, 2H), 1.76 (m, 2H), 1.55 (m, 2H), 1.35 (m, 2H), 1.16 (t, 3H), 0.97 (t, 3H).

3-[Butyl-pyridin-2-yl-amino]-propan-1-ol (5c). ¹H NMR (400 MHz, CDCl₃), δ (ppm): 8.01 (d, 1H), 7.40 (m, 1H), 6.45 (m, 2H), 5.70 (s, 1H), 3.71 (t, 2H), 3.45 (t, 2H), 3.22 (t, 2H), 1.76 (m, 2H), 1.65 (m, 2H), 1.35 (m, 2H), 0.97 (t, 3H).

3-[Benzothiazol-2-yl-butyl-amino]-propan-1-ol (5d). ¹H NMR (400 MHz, CDCl₃), δ (ppm): 7.55 (d, 1H), 7.47 (d, 1H), 7.26 (m, 1H), 6.98 (t, 1H), 5.29 (br, 1H), 3.82 (t, 2H), 3.55 (m, 2H), 3.32 (t, 2H), 1.80 (m, 2H), 1.71 (m, 2H), 1.39 (m, 2H), 0.97 (t, 3H).

3-[Benzooxazol-2-yl-butyl-amino]-propan-1-ol (5e). ¹H NMR (400 MHz, CDCl₃), δ (ppm): 7.31 (d, 1H), 7.28 (d, 1H), 7.13 (m, 1H), 6.98 (t, 1H), 5.00 (br, 1H), 3.72 (t, 2H), 3.59 (m, 2H), 3.47 (t, 2H), 1.82 (m, 2H), 1.69 (m, 2H), 1.40 (m, 2H), 0.99 (t, 3H).

3-[Benzothiazol-2-yl-(4-trifluoromethyl-benzyl)-amino]-propan-1-ol (5f). ¹H NMR (400 MHz, CDCl₃) δ ppm: 7.61 (d, 2H), 7.55 (d, 2H), 7.42 (d, 2H), 7.32 (t, 1H), 7.10 (t, 1H), 4.69 (s, 2H), 3.82 (t, 2H), 3.60 (t, 2H), 1.79 (m, 2H).

3-[Benzothiazol-2-yl-(2,4-bis-trifluoromethyl-benzyl)-amino]-propan-1-ol (5g). ¹H NMR (400 MHz, CDCl₃) δ ppm: 7.98 (s, 1H), 7.76 (d, 1H), 7.56 (m, 3H), 7.31 (t, 1H), 7.11 (t, 1H), 4.89 (s, 2H), 3.77 (t, 2H), 3.62 (t, 2H), 1.84 (m, 2H).

3-[(Benzooxazol-2-yl)-(2,4-bis-trifluoromethyl-benzyl)-amino]-propan-1-ol (5h). ¹H NMR (400 MHz, CDCl₃), δ (ppm): 7.97 (s, 1H), 7.78 (d, 1H), 7.54 (d, 1H), 7.38 (d, 1H), 7.24 (m, 2H), 7.07 (m, 1H), 5.00 (s, 2H), 4.37 (br, 1H), 3.77 (m, 2H), 3.66 (m, 2H), 1.85 (m, 2H).

3-[(2,4-Bis-trifluoromethyl-benzyl)-(pyrid-2-yl)-amino]-propan-1-ol (5i). ¹H NMR (400 MHz, CDCl₃), δ (ppm): 7.97 (s, 1H), 7.67 (d, 1H), 7.44 (d, 1H), 6.91 (d, 1H), 6.61 (t, 2H), 6.50 (d, 1H), 5.04 (s, 2H), 4.37 (br, 1H), 3.73 (m, 2H), 3.66 (m, 2H), 1.85 (m, 2H).

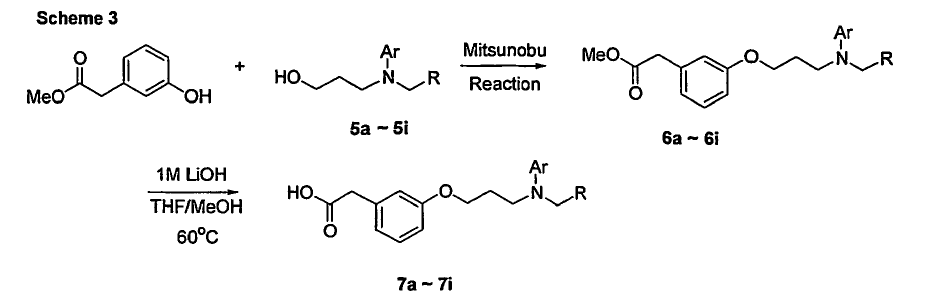

| | **R** | **Ar** |
|---|---|---|
| **7a** | 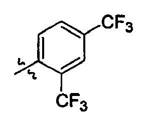 | 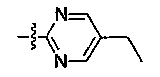 |
| **7b** | 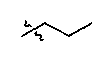 | 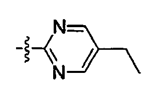 |
| **7c** | 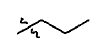 | 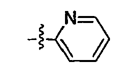 |
| **7d** | 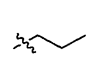 | 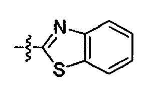 |
| **7e** | 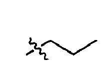 | 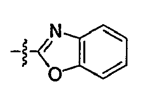 |
| **7f** | 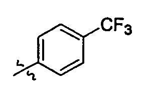 | 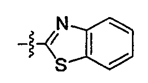 |
| **7g** | 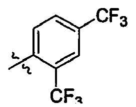 | 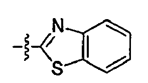 |
| **7h** | 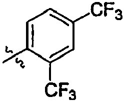 | 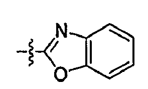 |
| **7i** | 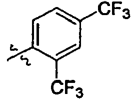 | 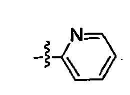 |

### EXAMPLE 3A: Synthesis of (3-{3-[Butyl-(5-ethyl-pyrimidin-2-yl)-amino]-propoxy}-phenyl)-acetic acid (7b) (Scheme 3).

Alcohol (5b) (162 mg, 0.69 mmol) and triphenylphosphine (218 mg, 0.83 mmol) were dissolved in 5 mL of ether followed by addition of methyl 3-hydroxyphenylacetate (115 mg, 0.69 mmol). The resulting solution was cooled to 0°C and then was added diisopropyl azodicarboxylate (163 µL, 0.83 mmol) in three potions with stirring. After stirring for 10 min at same temperature, the reaction mixture was warmed up to rt and stirred overnight. The resulting precipitate was filtered out through a pad of silica gel and the organic solution was concentrated. The residue was purified by chromatography to give 11mg (4% yield) of desired ester (**6b**), which was hydrolyzed with 1N LiOH (60 µL, 0.06 mmol) in 2 mL THF/MeOH (3:1) solution to give 9.0 mg product (**7b**). ¹H NMR (400 MHz, CDCl₃), δ (ppm): 8.16 (s, 2H), 7.22 (m, 1H), 6.81 (m, 3H), 3.99 (t, 2H), 3.70 (t, 2H), 3.61 (s, 2H), 3.54 (t, 2H), 2.43 (q, 2H), 2.10 (m, 2H), 1.59 (m, 2H), 1.31 (m, 2H), 1.18 (t, 3H), 0.89 (t, 3H).

### Example 3B. Synthesis of Compounds 7a and 7c - 7i followed the same procedure as described for the compound 7b (see Scheme 3).

(3-{3-[(2,4-Bis-trifluoromethyl-benzyl)-(5-ethyl-pyrimidin-2-yl)-amino]-propoxy}-phenyl)-acetic acid (7a). ¹H NMR (400 MHz, CDCl₃), δ (ppm): 8.18 (s, 2H), 7.90 (s, 1H), 7.67 (d, 1H), 7.38 (d, 1H), 7.21 (t, 1H), 6.85 (t, 1H), 6.76 (m, 2H), 5.11 (s, 2H), 4.00 (t, 2H), 3.78 (t, 2H), 3.60 (s, 2H), 2.47 (q, 2H), 2.15 (m, 2H), 1.21 (t, 3H).
{3-[3-(Butyl-pyridin-2-yl-amino)-propoxy]-phenyl}-acetic acid (7c). ¹H NMR (400 MHz, CDCl₃), δ (ppm): 8.11 (m, 1H), 7.39 (m, 1H), 7.22 (m, 1H), 6.85 (m, 3H), 6.49 (m, 2H), 3.98 (t, 2H), 3.71 (t, 2H), 3.67 (s, 2H), 3.42 (t, 2H), 2.10 (m, 2H), 1.55 (m, 2H), 1.31 (m, 2H), 0.89 (t, 3H).
{3-[3-(Benzothiazol-2-yl-butyl-amino)-propoxy]-phenyl}-acetic acid (7d). ¹H NMR (400 MHz, CDCl₃), δ (ppm): 7.51 (d, 1H), 7.46 (d, 1H), 7.23 (m, 2H), 7.00 (t, 1H), 6.78 (m, 2H), 4.00 (t, 2H), 3.73 (t, 2H), 3.57 (s, 2H), 3.45 (t, 2H), 2.16 (m, 2H), 1.66 (m, 2H), 1.37 (m, 2H), 0.89 (t, 3H).
{3-[3-(Benzooxazol-2-yl-butyl-amino)-propoxy]-phenyl}-acetic acid (7e). ¹H NMR (400 MHz, CDCl₃), δ (ppm): 7.36 (d, 1H), 7.21 (m, 2H), 7.14 (t, 1H), 6.98 (t, 1H), 6.84 (m, 2H), 4.02 (t, 2H), 3.72 (t, 2H), 3.61 (s, 2H), 3.52 (t, 2H), 2.17 (m, 2H), 1.66 (m, 2H), 1.37 (m, 2H), 0.90 (t, 3H).
(3-{3-[Benzothiazol-2-yl-(4-trifluoromethyl-benzyl)-amino]-propoxy}-phenyl)-acetic acid (7f). ¹H NMR (400 MHz, CDCl₃), δ ppm: 7.59 (d, 4H), 7.41 (d, 2H), 7.31 (t, 1H), 7.24 (t, 1H), 7.10 (t, 1H), 6.89 (d, 1H), 6.83 (s, 1H), 6.79 (d, 1H), 4.88 (s, 2H), 4.02 (t, 2H), 3.73 (t, 2H), 3.63 (s, 2H), 2.21 (m, 2H).
(3-{3-[Benzothiazol-2-yl-(2,4-bis-trifluoromethyl-benzyl)-amino]-propoxy]-acetic acid (7g). ¹H NMR (400 MHz, CDCl₃), δ ppm: 7.92 (s, 1H), 7.72 (d, 1H), 7.59 (m, 3H), 7.31 (t, 1H), 7.24 (t, 1H), 7.10 (t, 1H), 6.86 (d, 1H), 6.80 (s, 1H), 6.79 (d, 1H), 5.11 (s, 2H), 4.03 (t, 2H), 3.74 (t, 2H), 3.59 (s, 2H), 2.22 (m, 2H).
(3-{3-[Benzooxazol-2-yl-(2,4-bis-trifluoromethyl-benzyl)-amino]-propoxy}-phenl)-acetic acid (7h). ¹H NMR (400 MHz, CDCl₃), δ ppm: 7.92 (s, 1H), 7.72 (d, 1H), 7.57 (d, 1H), 7.39 (d, 1H), 7.20 (m, 3H), 7.04 (t, 1H), 6.86 (d, 1H), 6.76 (m, 2H), 5.07 (s, 2H), 4.03 (t, 2H), 3.76 (t, 2H), 3.59 (s, 2H), 2.20 (m, 2H).
(3-{3-[(2,4-Bis-trifluoromethyl-benzyl)-pyridin-2-yl)-amino]-propoxy}-phenyl)-acetic acid (7i). ¹H NMR (400 MHz, CDCl₃), δ (ppm): 8.18 (m, 1H), 7.91 (s, 1H), 7.67 (d, 1H), 7.40 (m, 2H), 7.23 (t, 1H), 6.87 (d, 1H), 6.79 (m, 2H), 6.60 (m, 1H), 6.49 (d, 2H), 5.02 (s, 2H), 4.01 (t, 2H), 3.72 (t, 2H), 3.41 (s, 2H), 2.15 (m, 2H).

### EXAMPLES 4: The following naphthyl derivatives were synthesized in a manner analogous to that described for Example 3A using the appropriate hydroxynaphthyl acetate.

### 1-{3-[(2,4-Bis-trifluoromethyl-benzyl)-(5-ethyl-pyrimidin-2-yl)-amino]-propoxy}-naphthalene-2-carboxylic acid (8a).

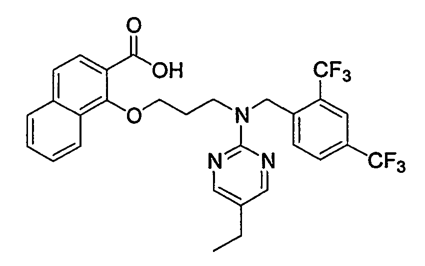

¹H NMR (400 MHz, CDCl₃), δ (ppm): 8.22 (s, 2H), 8.14 (m, 2H), 7.97 (m, 2H), 7.72 (m, 2H), 7.62 (m, 2H), 7.45 (d, 1H), 5.21 (s, 2H), 4.33 (t, 2H), 3.95 (t, 2H), 2.55 (q, 2H), 2.39 (m, 2H), 1.23 (t, 3H).

### 6-{3-[(2,4-Bis-trifluoromethyl-benzyl)-(5-ethyl-pyrimidin-2-yl)-amino]-propoxy}-naphthalene-2-carboxylic acid (8b).

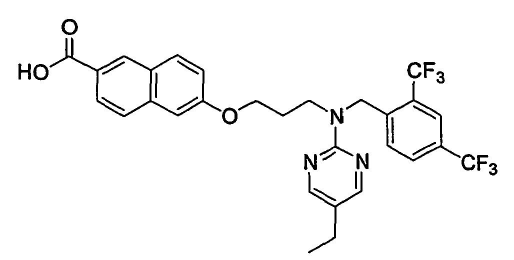

¹H NMR (400 MHz, CDCl₃), δ (ppm): 8.52 (s, 1H), 8.21 (s, 2H), 8.12 (d, 1H), 7.91 (s, 1H), 7.82 (d, 1H), 7.73 (m, 2H), 7.40 (d, 1H), 7.11 (m, 2H), 5.15 (s, 2H), 4.13 (t, 2H), 3.85 (t, 2H), 2.46 (q, 2H), 2.25 (m, 2H), 1.19 (t, 3H).

### 3-{3-[(2,4-Bis-trifluoromethyl-benzyl)-(5-ethyl-pyrimidin-2-yl)-amino]-propoxy}-naphthalene-2-carboxylic acid (8c).

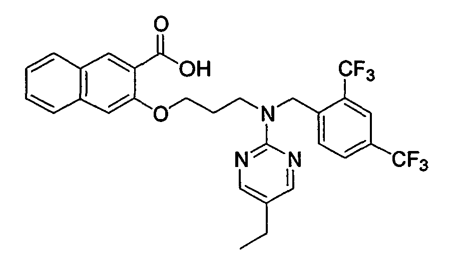

¹H NMR (400 MHz, CDCl₃), δ (ppm): 8.73 (s, 1H), 8.09 (s, 2H), 7.91 (m, 2H), 7.71 (m, 2H), 7.52 (t, 1H), 7.41 (m, 2H), 7.15 (s, 1H), 5.15 (s, 2H), 4.31 (t, 2H), 3.89 (t, 2H), 2.39 (q, 2H), 2.27 (m, 2H), 1.05 (t, 3H).

### 1-{3-[Butyl-(5-ethyl-pyrimidin-2-yl)-amino]-propoxy}-naphthalene-2-carboxylic acid (8d).

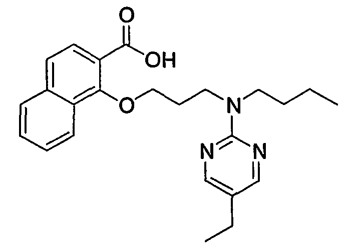

¹H NMR (400 MHz, CDCl₃), δ (ppm): 8.16 (s, 2H), 8.15 (s, 1H), 8.06 (d, 1H), 7.89 (d, 1H), 7.69 (d, 1H), 7.60 (m, 2H), 4.33 (t, 2H), 3.87 (t, 2H), 3.59 (t, 2H), 2.44 (q, 2H), 2.32 (m, 2H), 1.61 (m, 2H), 1.35 (m, 2H), 1.16 (t, 3H), 0.95 (t, 3H).

### 1-[3-(Butyl-pyridin-2-yl-amino)-propoxy]-naphthalene-2-carboxylic acid (8e).

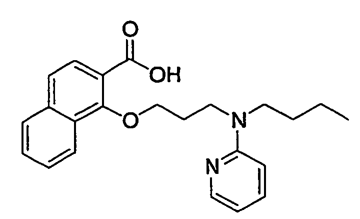

¹H NMR (400 MHz, CDCl₃), δ (ppm): 8.14 (s, 1H), 8.13 (d, 1H), 8.00 (d, 1H), 7.85 (d, 1H), 7.63 (d, 1H), 7.54 (m, 2H), 7.45 (m, 1H), 6.52 (m, 2H), 4.31 (t, 2H), 3.88 (t, 2H), 3.43 (t, 2H), 2.27 (m, 2H), 1.61 (m, 2H), 1.36 (m, 2H), 0.95 (t, 3H).

### 1-{3-(Benzothiazol-2-yl-butyl-amino)-propoxy]-naphtalene-2-carboxylic acid (8f).

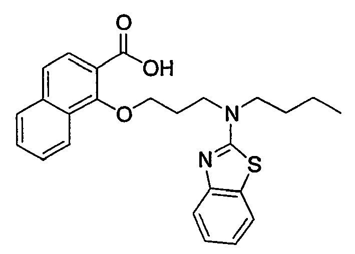

¹H NMR (400 MHz, CDCl₃) δ ppm. 8.18 (d, 1H), 7.78 (d, 1H), 7.53 (d, 2H), 7.26 (m, 3H), 7.60 (d, 1H), 7.40 (t, 1H), 7.85 (t, 1H), 4.05 (t, 2H), 3.72 (t, 2H), 3.42 (t, 2H), 2.30 (t, 2H), 1.59 (t, 2H), 1.25 (q, 2H), 0.88 (t, 3H).

### 1-{3-(Benzothiazol-2-yl-(4-trifluoromethyl-benyl)amino]-propoxy}-naphthalene-2-carboxylic acid (8g).

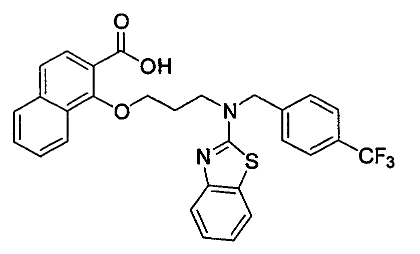

¹H NMR (400 MHz, CDCl₃) δ ppm. 8.14 (d, 1H), 8.00 (d, 1H), 7.86 (d, 1H), 7.57-7.67 (m, 6H), 7.50 (t, 1H), 7.46 (d, 2H), 7.31 (t, 1H), 7.10 (t, 1H), 4.91 (s, 2H), 4.30 (t, 2H), 3.96 (t, 2H), 2.39 (t, 2H).

### 1-[3-(Benzooxazol-2-yl-butyl-amino)-propoxy]-naphthalene-2-carboxylic acid (8h).

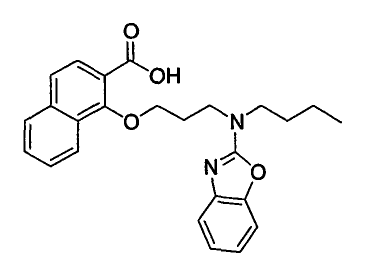

¹H NMR (400 MHz, CDCl₃), δ (ppm): 8.16 (d, 1H), 7.99 (d, 1H), 7.86 (d, 1H), 7.65 (d, 1H), 7.59 (t, 1H), 7.52 (t, 1H), 7.41 (d, 1H), 7.26 (d, 1H), 7.16 (t, 1H), 7.01 (t, 1H), 4.32 (t, 2H), 3.92 (t, 2H), 3.58 (t, 2H), 2.36 (m, 2H), 1.70 (m, 2H), 1.40 (m, 2H), 0.97 (t, 3H).

### 1-{3-[Benzooxazol-2-yl-(2,4-bis-trifluoromethyl-benzyl)-amino]-propoxy}-naphthalene-2-carboxylic acid (8i).

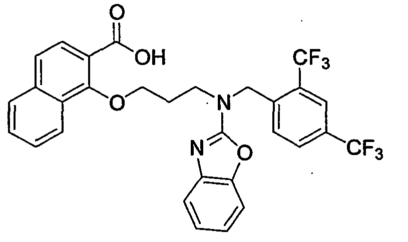

¹H NMR (400 MHz, CDCl₃), δ (ppm): 8.23 (d, 1H), 8.17 (m, 1H), 7.94 (s, 1H), 7.86 (s,1H), 7.84 (s, 1H), 7.70 (d, 1H), 7.59 (m, 2H), 7.49 (m, 3H), 6.62 (dt, 1H), 6.56 (d, 1H), 5.10 (s, 2H), 4.23 (t, 2H), 3.93 (t, 2H), 2.32 (m, 2H).

### Example 5: Biological activity.

The compounds were evaluated in a cell-based assay to determine their human PPAR activity. The plasmids for human PPAR-GAL4 chimeras were prepared by fusing amplified cDNAs encoding the LBDs of PPARs to the C-terminal end of the yeast GAL4 DNA binding domain. CV-1 cells were grown and transiently tranfected with PerFectin (GTS, San Diego, CA) according to the manufacturer's protocol along with a luciferase reporter. Eight hours after transfection, 50 µL of cells were replated into 384 well plates (1X10⁵ cells/well). Sixteen hours after replating, cells were treated with either compounds or 1% DMSO for 24 hours. Luciferase activity was then assayed with Britelite (Perkin Elmer) following the manufacturer's protocol and measured with either the Perkin Elmer Viewlux or Molecular Devices Acquest.

**Table: Activity of Compounds**

| **Compound** | **hPPAP α** **(EC**_{**50**}**)** **A > 100µM** **B = 100-1 µM** **C = < 1 µM** | **hPPAR γ** **(EC**_{**50**}**)** **A > 100µM** **B = 100-1 µM** **C = < 1 µM** | **hPPARδ** **(EC**_{**50**}**) A > 100µM** **B = 100-1 µM** **C = < 1 µM** |
|---|---|---|---|
| 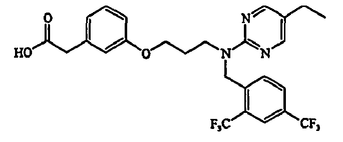 | C | C | C |
| 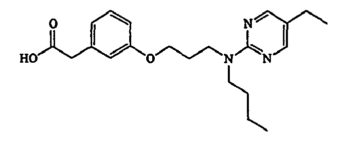 | A | C | B |
| 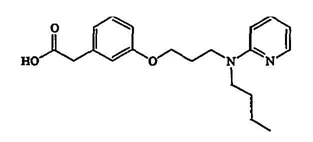 | A | C | B |
| 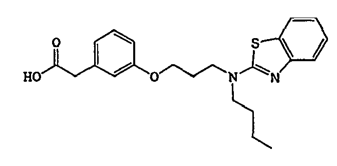 | B | A | B |
| 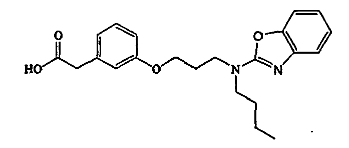 | B | A | B |
| 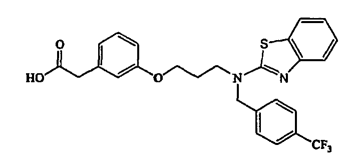 | C | C | C |
| 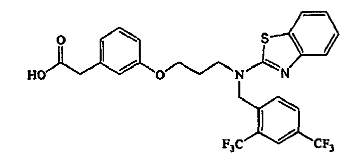 | A | C | B |
| 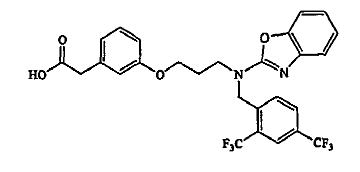 | A | C | B |
| 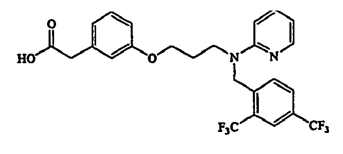 | A | C | C |
| 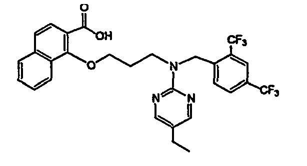 | B | C | A |
| 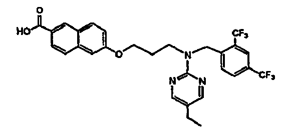 | A | A | A |
| 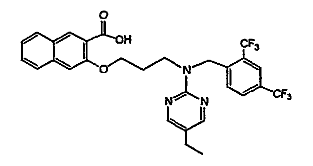 | B | C | A |
| 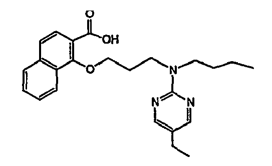 | A | C | A |
| 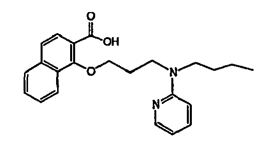 | A | C | A |
| 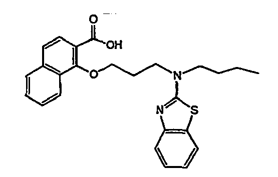 | A | B | B |
| 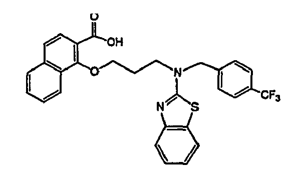 | A | C | A |
| 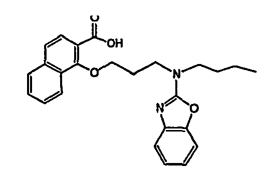 | A | B | A |
| 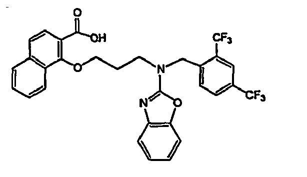 | A | C | A |

### Examples of Pharmaceutical Formulations

As a guide only, the compounds of Formula (I) may be formulated into pharmaceutical compositions according to the following general examples.

### Parenteral Composition

To prepare a parenteral pharmaceutical composition suitable for administration by injection, 100 mg of a water-soluble salt of a compound of Formula (I) is dissolved in DMSO and then mixed with 10 mL of 0.9% sterile saline. The mixture is incorporated into a dosage unit form suitable for administration by injection.

### Oral Composition

To prepare a pharmaceutical composition for oral delivery, 100 mg of a compound of Formula I is mixed with 750 mg of lactose. The mixture is incorporated into an oral dosage unit for, such as a hard gelatin capsule, which is suitable for oral administration.

Those of skill in the art will appreciate that the compounds and uses disclosed herein can be used as PPAR modulators, providing a therapeutic effect.

One skilled in the art will appreciate that these methods and compounds are and may be adapted to carry out the objects and obtain the ends and advantages mentioned, as well as those inherent therein. The methods, procedures, and compounds described herein are exemplary and are not intended as limitations on the scope of the invention. Changes therein and other uses will occur to those skilled in the art which are encompassed within the spirit of the invention and are defined by the scope of the claims.

It will be apparent to one skilled in the art that varying substitutions and modifications may be made to the invention disclosed herein without departing from the scope and spirit of the invention.

Those skilled in the art recognize that the aspects and embodiments of the invention set forth herein may be practiced separate from each other or in conjunction with each other. Therefore, combinations of separate embodiments are within the scope of the invention as claimed herein.

All patents and publications mentioned in the specification are indicative of the levels of those skilled in the art to which the invention pertains. All patents and publications are herein incorporated by reference to the same extent as if each individual publication was specifically and individually indicated to be incorporated by reference.

The invention illustratively described herein may be practiced in the absence of any element or elements, limitation or limitations which is not specifically disclosed herein. Thus, for example, in each instance herein any of the terms "comprising", "consisting essentially of" and "consisting of" may be replaced with either of the other two terms. The terms and expressions which have been employed are used as terms of description and not of limitation, and there is no intention that the use of such terms and expressions indicates the exclusion of equivalents of the features shown and described or portions thereof. It is recognized that various modifications are possible within the scope of the invention claimed. Thus, it should be understood that although the present invention has been specifically disclosed by certain embodiments and optional features, modification and variation of the concepts herein disclosed may be resorted to by those skilled in the art, and that such modifications and variations are considered to be within the scope of this invention as defined by the appended claims.

In addition, where features or aspects of the invention are described in terms of Markush groups, those skilled in the art will recognize that the invention is also thereby described in terms of any individual member or subgroup of members of the Markush group. For example, if X is described as selected from the group consisting of bromine, chlorine, and iodine, claims for X being bromine and claims for X being bromine and chlorine are fully described.

Other embodiments are within the following claims.

## Claims

1. A compound having the structure of Formula I: 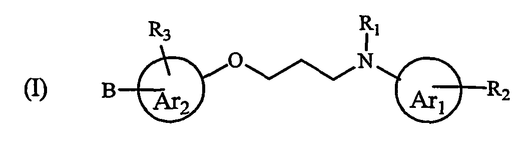 wherein
Ar₁ is selected from the group consisting of a monocyclic heteroaromatic ring structure and a bicyclic heteroaromatic ring structure;
Ar₂ is selected from the group consisting of a monocyclic, a bicyclic, and a tricyclic carbocyclic aryl ring structure
R₁ is selected from the group consisting of
alkyl, optionally substituted with a substituent selected from the group consisting of hydrogen, lower alkyl, optionally substituted carbocyclic or heterocyclic ring, halogen, perhaloalkyl, hydroxy, alkoxy, nitro, and amino;
a five-membered or six-membered heteroaryl ring or a six-membered aryl ring, optionally substituted with one or more substituents selected from the group consisting of optionally substituted C₁-C₈ straight-chain, branched, or cyclic saturated or unsaturated alkyl; an alkoxy; cyano; nitro; an amino; an amido; perhaloalkyl; and halogen;
R₂ is selected from the group consisting of
hydrogen;
alkyl, optionally substituted with a substituent selected from the group consisting of hydrogen, lower alkyl, optionally substituted carbocyclic or heterocyclic ring, halogen, perhaloalkyl, hydroxy, alkoxy, nitro, and amino;
a five-membered or six-membered heteroaryl ring or a six-membered aryl ring, optionally substituted with one or more substituents selected from the group consisting of optionally substituted C₁-C₈ straight-chain, branched, or cyclic saturated or unsaturated alkyl; an alkoxy; halogen; and perhaloalkyl;
cyano; nitro; an amino; an amido; perhaloalkyl; and halogen;
R₃ is selected from the group consisting of hydrogen; alkyl, optionally substituted with a substituent selected from the group consisting of hydrogen, lower alkyl, optionally substituted carbocyclic or heterocyclic ring; hydroxy; halogen; amino; nitro; and cyano; and
B is a five-membered or six-membered heteroaryl ring, or -(CH₂)ⱼ-C(O)OR₄, wherein j is
0 or 1 when Ar₂ is a bicyclic or tricyclic carbocyclic ring structure and j is 1 when Ar₂ is a monocyclic carbocyclic ring structure; and
R₄ is selected from the group consisting of
hydrogen;
alkyl, optionally substituted with a substituent selected from the group consisting of hydrogen, lower alkyl, optionally substituted carbocyclic or heterocyclic ring;
a five-membered or six-membered heteroaryl ring or a six-membered aryl ring, optionally substituted with one or more substituents selected from the group consisting of optionally substituted C₁-C₈ straight-chain, branched, or cyclic saturated or unsaturated alkyl;
or a pharmaceutically acceptable N-oxide, pharmaceutically acceptable prodrug, pharmaceutically active metabolite, pharmaceutically acceptable salt, pharmaceutically acceptable ester, pharmaceutically acceptable amide, or pharmaceutically acceptable solvate thereof.

2. The compound of Claim 1, wherein Ar₂ is selected from the group consisting of phenyl, naphthyl, anthracene, and phenanthrene.

3. The compound of Claim 2, wherein Ar₂ is phenyl.

4. The compound of Claim 3, having the structure:

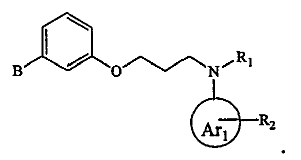

5. The compound of claim 2, wherein Ar₂ is naphthyl.

6. The compound of Claim 4 or Claim 5, wherein R₁ is alkyl, optionally substituted with one or more optionally substituted carbocyclic or heterocyclic rings.

7. The compound of Claim 6, wherein said alkyl is a lower alkyl.

8. The compound of Claim 7, wherein said lower alkyl is selected from the group consisting of methyl, ethyl, n-propyl, isopropyl, n-butyl, tert-butyl, and sec-butyl.

9. The compound of Claim 6, wherein said carbocyclic ring is phenyl.

10. The compound of Claim 9, wherein said phenyl is optionally substituted with one or more substituents selected from the group consisting of lower alkyl, halogen, perhaloalkyl, hydroxy, alkoxy, nitro, and amino.

11. The compound of Claim 10, wherein said substituent is perhaloalkyl.

12. The compound of Claim 11, wherein said perhaloalkyl is trifluoromethyl.

13. The compound of Claim 1, wherein R₁ is alkyl substituted with 4-bis(trifluoromethyl)phenylmethyl.

14. The compound of Claim 1, wherein Ar₁ is a nitrogen-containing or oxygen-containing heterocycle.

15. The compound of Claim 14, wherein Ar₁ is selected from the group consisting of furan, thiophene, pyrrole, pyrroline, pyrrolidine, oxazole, thiazole, imidazole, imidazoline, imidazolidine, pyrazole, pyrazoline, pyrazolidine, isoxazole, isothiazole, triazole, tetrazole, thiadiazole, pyran, pyridine, piperidine, morpholine, thiomorpholine, pyridazine, pyrimidine, pyrazine, piperazine, triazine,

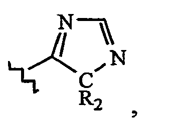

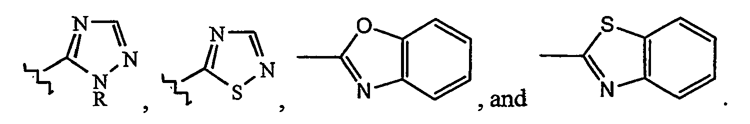

16. The compound of Claim 15, wherein Ar₁ is pyridine, pyrimidine,

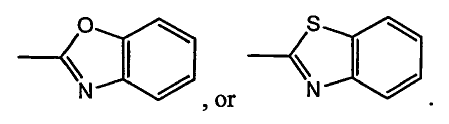

17. The compound of Claim 16, wherein Ar₁ is pyrimidine.

18. The compound of any of Claim 4 and Claim 5, wherein R₂ is optionally substituted alkyl.

19. The compound of Claim 18, wherein said alkyl is a lower alkyl.

20. The compound of Claim 19, wherein said lower alkyl is selected from the group consisting of methyl, ethyl, n-propyl, isopropyl, n-butyl, tert-butyl, and sec-butyl.

21. The compound of any of Claim 20, wherein R₂ is ethyl.

22. The compound of Claim 1, wherein R₃ is hydrogen, halogen or optionally substituted alkyl.

23. The compound of Claim 22, wherein said optionally substituted alkyl is an optionally substituted lower alkyl.

24. The compound of Claim 23, wherein said optionally substituted lower alkyl is selected from the group consisting of methyl, ethyl, n-propyl, isopropyl, n-butyl, tert-butyl, and sec-butyl.

25. The compound of Claim 1, wherein R₃ is methyl.

26. The compound of Claim 1, wherein R₃ is hydrogen.

27. The compound of Claim 1, wherein B and the propyloxy substituents on Ar₂ are ortho to each other.

28. The compound of Claim 1, wherein B and the propyloxy substituents on Ar₂ are meta to each other.

29. The compound of Claim 1, wherein B and the propyloxy substituents on Ar₂ are para to each other.

30. The compound of Claim 1, wherein B is a heteroaryl ring selected from the group consisting of furan, thiophene, pyrrole, pyrroline, pyrrolidine, oxazole, thiazole, imidazole, imidazoline, imidazolidine, pyrazole, pyrazoline, pyrazolidine, isoxazole, isothiazole, triazole, tetrazole, thiadiazole, pyran, pyridine, piperidine, morpholine, thiomorpholine, pyridazine, pyrimidine, pyrazine, piperazine, triazine,

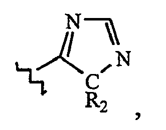

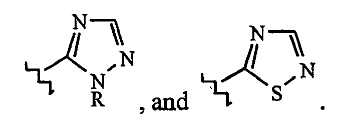

31. The compound of Claim 30, wherein B is a tetrazole.

32. The compound of Claim 1, wherein B is -(CH₂)ⱼ-C(O)OR₄.

33. The compound of Claim 32, wherein R₄ is hydrogen or optionally substituted alkyl.

34. The compound of Claim 33, wherein said alkyl is a lower alkyl.

35. The compound of Claim 34, wherein said lower alkyl is selected from the group consisting of methyl, ethyl, n-propyl, isopropyl, n-butyl, tert-butyl, and sec-butyl.

36. The compound of Claim 33, wherein R₄ is hydrogen.

37. The compound of any of Claim 4 and Claim 5, wherein Ar₁ is a nitrogen-containing or oxygen-containing heterocycle.

38. The compound of Claim 37, wherein Ar₁ is selected from the group consisting of furan, thiophene, pyrrole, pyrroline, pyrrolidine, oxazole, thiazole, imidazole, imidazoline, imidazolidine, pyrazole, pyrazoline, pyrazolidine, isoxazole, isothiazole, triazole, tetrazole, thiadiazole, pyran, pyridine, piperidine, morpholine, thiomorpholine, pyridazine, pyrimidine, pyrazine, piperazine, triazine,

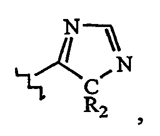

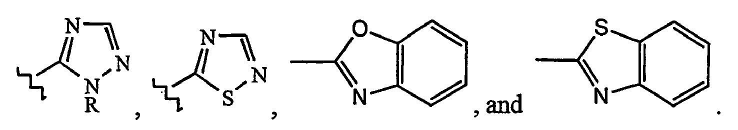

39. The compound of Claim 38, wherein Ar₁ is pyridine, pyrimidine,

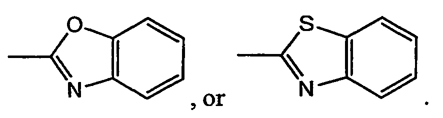

40. The compound of Claim 39, wherein Ar₁ is pyrimidine.

41. The compound of any of Claim 4 and Claim 5, wherein B is a heteroaryl ring selected from the group consisting of furan, thiophene, pyrrole, pyrroline, pyrrolidine, oxazole, thiazole, imidazole, imidazoline, imidazolidine, pyrazole, pyrazoline, pyrazolidine, isoxazole, isothiazole, triazole, tetrazole, thiadiazole, pyran, pyridine, piperidine, morpholine, thiomorpholine, pyridazine, pyrimidine, pyrazine, piperazine, triazine,

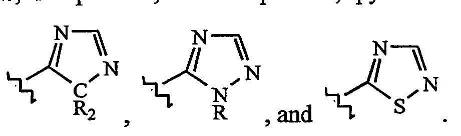

42. The compound of Claim 41, wherein B is a tetrazole.

43. The compound of any of Claim 4 and Claim 5, wherein B is -(CH₂)ⱼ₋C(O)OR₄.

44. The compound of Claim 43, wherein R₄ is hydrogen or optionally substituted alkyl.

45. The compound of Claim 44, wherein said alkyl is a lower alkyl.

46. The compound of Claim 45, wherein said lower alkyl is selected from the group consisting of methyl, ethyl, n-propyl, isopropyl, n-butyl, tert-butyl, and sec-butyl.

47. The compound of any of Claim 4 and Claim 5, wherein R₄ is hydrogen.

48. The compound of Claim 4 selected from the group consisting of: 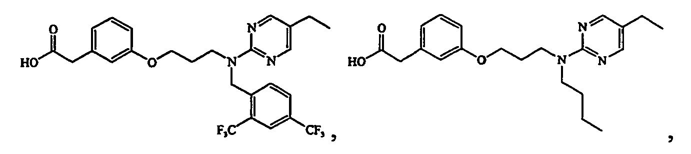 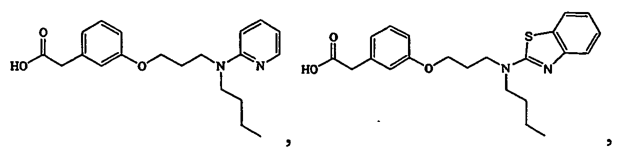 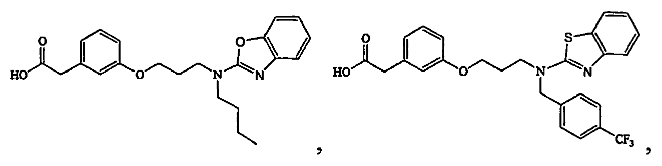 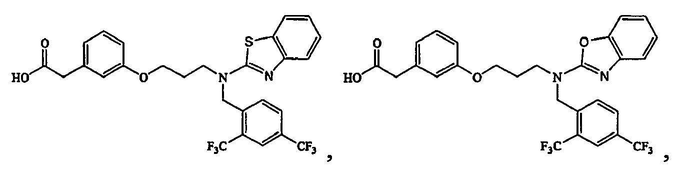 and 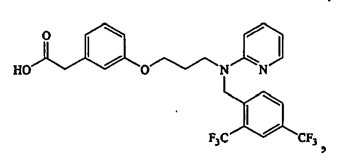 or a pharmaceutically acceptable N-oxide, pharmaceutically acceptable prodrug, pharmaceutically active metabolite, pharmaceutically acceptable salt, pharmaceutically acceptable ester, pharmaceutically acceptable amide, or pharmaceutically acceptable solvate thereof.

49. The compound of Claim 5 selected from the group consisting of 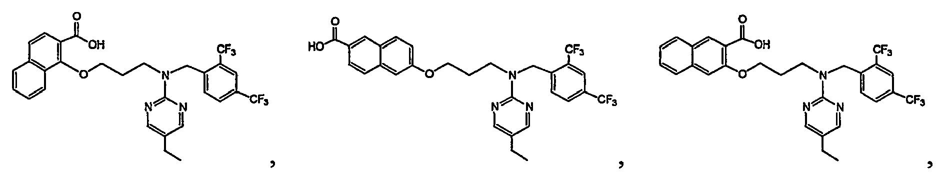 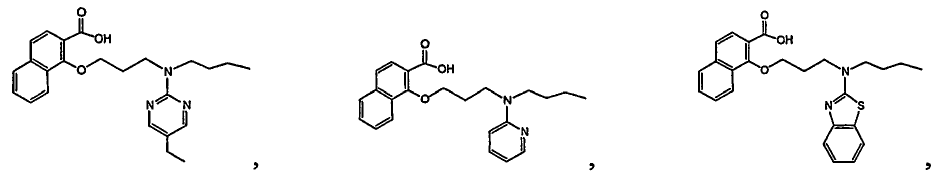 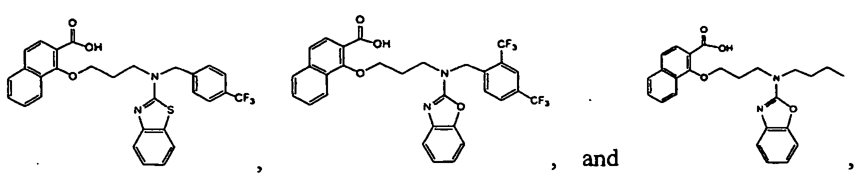 or a pharmaceutically acceptable N-oxide, pharmaceutically acceptable prodrug, pharmaceutically active metabolite, pharmaceutically acceptable salt, pharmaceutically acceptable ester, pharmaceutically acceptable amide, or pharmaceutically acceptable solvate thereof.

50. A compound having the structure of Formula III:

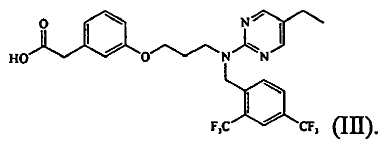

51. Use of a compound as claimed in Claims 1 to 50 for the preparation of a medicament for the prophylactic or therapeutic treatment of a peroxisome proliferator-activated receptor (PPAR)-modulated disease or condition.

52. The use of Claim 51, wherein the disease is a metabolic disorder or condition.

53. The use of Claim 51, wherein said disease is selected from the group consisting of obesity, diabetes, hyperinsulinemia, metabolic syndrome X, polycystic ovary syndrome, climacteric, disorders associated with oxidative stress, inflammatory response to tissue injury, pathogenesis of emphysema, ischemia-associated organ injury, doxorubicin-induced cardiac injury, drug-induced hepatotoxicity, atherosclerosis, and hypertoxic lung injury.

54. A pharmaceutical composition comprising a compound of Claims 1 to 50 and a pharmaceutically acceptable diluent, excipient, or carrier.

## Patentansprüche

1. Verbindung mit der Struktur der Formel I: 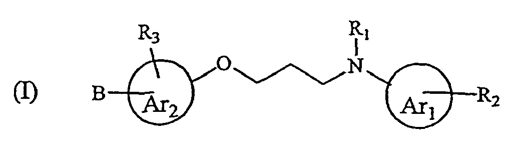 worin
Ar₁ ausgewählt ist aus der Gruppe, bestehend aus einer monocyclischen heteroaromatischen Ringstruktur und einer bicyclischen heteroaromatischen Ringstruktur;
Ar₂ ausgewählt ist aus der Gruppe, bestehend aus einer monocyclischen, einer bicyclischen und einer tricyclischen carbocyclischen Arylringstruktur;
R₁ ausgewählt ist aus der Gruppe, bestehend aus
Alkyl, gegebenenfalls substituiert mit einem Substituenten, ausgewählt aus der Gruppe, bestehend aus Wasserstoff, Niederalkyl, einem gegebenenfalls substituierten carbocyclischen oder heterocyclischen Ring, Halogen, Perhalogenalkyl, Hydroxy, Alkoxy, Nitro und Amino;
einem fünfgliedrigen oder sechsgliedrigen Heteroarylring oder einem sechsgliedrigen Arylring, gegebenenfalls substituiert mit einem oder mehr Substituenten, ausgewählt aus der Gruppe, bestehend aus gegebenenfalls substituiertem, geradkettigen, verzweigten oder cyclischen, gesättigten oder ungesättigten C₁-C₈-Alkyl; Alkoxy; Cyano; Nitro; Amino; Amido; Perhalogenalkyl; sowie Halogen;
R₂ ausgewählt ist aus der Gruppe, bestehend aus
Wasserstoff;
Alkyl, gegebenenfalls substituiert mit einem Substituenten, ausgewählt aus der Gruppe, bestehend aus Wasserstoff, Niederalkyl, einem gegebenenfalls substituierten carbocyclischen oder heterocyclischen Ring, Halogen, Perhalogenalkyl, Hydroxy, Alkoxy, Nitro und Amino;
einem fünfgliedrigen oder sechsgliedrigen Heteroarylring oder einem sechsgliedrigen Arylring, gegebenenfalls substituiert mit einem oder mehr Substituenten, ausgewählt aus der Gruppe, bestehend aus gegebenenfalls substituiertem, geradkettigen, verzweigten oder cyclischen, gesättigten oder ungesättigten C₁-C₈-Alkyl; Alkoxy; Halogen; und Perhalogenalkyl;
Cyano; Nitro; Amino; Amido; Perhalogenalkyl; und Halogen; R₃ ausgewählt ist aus der Gruppe, bestehend aus Wasserstoff; Alkyl, gegebenenfalls substituiert mit einem Substituenten, ausgewählt aus der Gruppe, bestehend aus Wasserstoff, Niederalkyl, einem gegebenenfalls substiuierten carbocyclischen oder heterocyclischen Ring; Hydroxy; Halogen; Amino; Nitro; und Cyano; und
B ein fünfgliedriger oder sechsgliedriger Heteroarylring oder -(CH₂)ⱼ-C(O)OR₄ ist, worin j für 0 oder 1 steht, wenn Ar₂ eine bicyclische oder tricyclische carbocyclische Ringstruktur ist, und j für 1 steht, wenn Ar₂ eine monocyclische carbocyclische Ringstruktur ist; und
R₄ ausgewählt ist aus der Gruppe, bestehend aus
Wasserstoff;
Alkyl, gegebenenfalls substituiert mit einem Substituenten, ausgewählt aus der Gruppe, bestehend aus Wasserstoff, Niederalkyl und einem gegebenenfalls substituierten carbocyclischen oder heterocyclischen Ring;
einem fünfgliedrigen oder sechsgliedrigen Heteroarylring oder einem sechsgliedrigen Arylring, gegebenenfalls substituiert mit einem oder mehr Substituenten, ausgewählt aus der Gruppe, bestehend aus gegebenenfalls substituiertem geradkettigen, verzweigten oder cyclischen, gesättigten oder ungesättigten C₁-C₈-Alkyl;
oder ein pharmazeutisch annehmbares N-Oxid, ein pharmazeutisch annehmbares Prodrug, ein pharmazeutisch aktiver Metabolit, ein pharmazeutisch annehmbares Salz, ein pharmazeutisch annehmbarer Ester, ein pharmazeutisch annehmbares Amid oder ein pharmazeutisch annehmbares Solvat davon.

2. Verbindung nach Anspruch 1, wobei Ar₂ ausgewählt ist aus der Gruppe, bestehend aus Phenyl, Naphthyl, Anthracen und Phenanthren.

3. Verbindung nach Anspruch 2, wobei Ar₂ Phenyl ist.

4. Verbindung nach Anspruch 3, die die folgende Struktur besitzt:

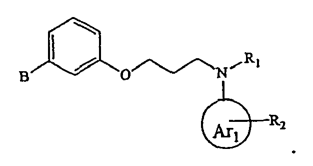

5. Verbindung nach Anspruch 2, wobei Ar₂ Naphthyl ist.

6. Verbindung nach Anspruch 4 oder Anspruch 5, wobei R₁ Alkyl ist, das gegebenenfalls mit einem oder mehr gegebenenfalls substituierten carbocyclischen oder heterocyclischen Ringen substituiert ist.

7. Verbindung nach Anspruch 6, wobei das Alkyl ein Niederalkyl ist.

8. Verbindung nach Anspruch 7, wobei das Niederalkyl ausgewählt ist aus der Gruppe, bestehend aus Methyl, Ethyl, n-Propyl, Isopropyl, n-Butyl, tert.-Butyl und sec.-Butyl.

9. Verbindung nach Anspruch 6, wobei der carbocyclische Ring Phenyl ist.

10. Verbindung nach Anspruch 9, wobei das Phenyl gegebenenfalls mit einem oder mehr Substituenten substituiert ist, die aus der Gruppe, bestehend aus Niederalkyl, Halogen, Perhalogenalkyl, Hydroxy, Alkoxy, Nitro und Amino, ausgewählt sind.

11. Verbindung nach Anspruch 10, wobei der Substituent Perhalogenalkyl ist.

12. Verbindung nach Anspruch 11, wobei das Perhalogenalkyl Trifluormethyl ist.

13. Verbindung nach Anspruch 1, wobei R₁ Alkyl ist, das mit 4-Bis(trifluormethyl)phenylmethyl substituiert ist.

14. Verbindung nach Anspruch 1, wobei Ar₁ ein Stickstoffenthaltender oder Sauerstoff-enthaltender Heterocyclus ist.

15. Verbindung nach Anspruch 14, wobei Ar₁ ausgewählt ist aus der Gruppe, bestehend aus Furan, Thiophen, Pyrrol, Pyrrolin, Pyrrolidin, Oxazol, Thiazol, Imidazol, Imidazolin, Imidazolidin, Pyrazol, Pyrazolin, Pyrazolidin, Isoxazol, Isothiazol, Triazol, Tetrazol, Thiadiazol, Pyran, Pyridin, Piperidin, Morpholin, Thiomorpholin, Pyridazin, Pyrimidin, Pyrazin, Piperazin, Triazin,

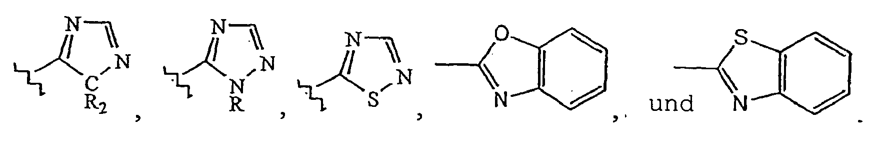

16. Verbindung nach Anspruch 15, wobei Ar₁ Pyridin, Pyrimidin, 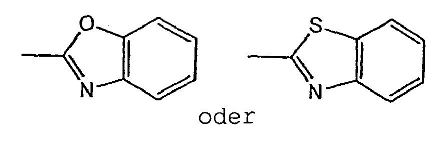 ist.

17. Verbindung nach Anspruch 16, wobei Ar₁ Pyrimidin ist.

18. Verbindung nach einem der Ansprüche 4 und 5, wobei R₂ gegebenenfalls substituiertes Alkyl ist.

19. Verbindung nach Anspruch 18, wobei das Alkyl ein Niederalkyl ist.

20. Verbindung nach Anspruch 19, wobei das Niederalkyl ausgewählt ist aus der Gruppe, bestehend aus Methyl, Ethyl, n-Propyl, Isopropyl, n-Butyl, tert.-Butyl und sec.-Butyl.

21. Verbindung nach Anspruch 20, wobei R₂ Ethyl ist.

22. Verbindung nach Anspruch 1, wobei R₃ Wasserstoff, Halogen oder gegebenenfalls substituiertes Alkyl ist.

23. Verbindung nach Anspruch 22, wobei das gegebenenfalls substituierte Alkyl ein gegebenenfalls substituiertes Niederalkyl ist.

24. Verbindung nach Anspruch 23, wobei das gegebenenfalls substituierte Niederalkyl ausgewählt ist aus der Gruppe, bestehend aus Methyl, Ethyl, n-Propyl, Isopropyl, n-Butyl, tert.-Butyl und sec.-Butyl.

25. Verbindung nach Anspruch 1, wobei R₃ Methyl ist.

26. Verbindung nach Anspruch 1, wobei R₃ Wasserstoff ist.

27. Verbindung nach Anspruch 1, wobei B und die Propyloxy-Substituenten an Ar₂ ortho zueinander stehen.

28. Verbindung nach Anspruch 1, wobei B und die Propyloxy-Substituenten an Ar₂ meta zueinander stehen.

29. Verbindung nach Anspruch 1, wobei B und die Propyloxy-Substituenten an Ar₂ para zueinander stehen.

30. Verbindung nach Anspruch 1, wobei B ein Heteroarylring ist, der aus der Gruppe, bestehend aus Furan, Thiophen, Pyrrol, Pyrrolin, Pyrrolidin, Oxazol, Thiazol, Imidazol, Imidazolin, Imidazolidin, Pyrazol, Pyrazolin, Pyrazolidin, Isoxazol, Isothiazol, Triazol, Tetrazol, Thiadiazol, Pyran, Pyridin, Piperidin, Morpholin, Thiomorpholin, Pyridazin, Pyrimidin, Pyrazin, Piperazin, Triazin, 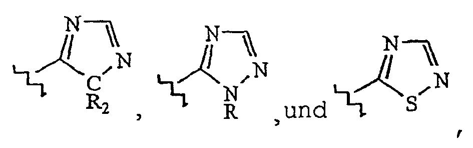 ausgewählt ist.

31. Verbindung nach Anspruch 30, wobei B ein Tetrazol ist.

32. Verbindung nach Anspruch 1, wobei B -(CH₂)ⱼ-C(O)OR₄ ist.

33. Verbindung nach Anspruch 32, wobei R₄ Wasserstoff oder gegebenenfalls substituiertes Alkyl ist.

34. Verbindung nach Anspruch 33, wobei das Alkyl ein Niederalkyl ist.

35. Verbindung nach Anspruch 34, wobei das Niederalkyl ausgewählt ist aus der Gruppe, bestehend aus Methyl, Ethyl, n-Propyl, Isopropyl, n-Butyl, tert.-Butyl und sec.-Butyl.

36. Verbindung nach Anspruch 33, wobei R₄ Wasserstoff ist.

37. Verbindung nach einem der Ansprüche 4 und 5, wobei Ar₁ ein Wasserstoff-enthaltender oder Sauerstoff-enthaltender Heterocyclus ist.

38. Verbindung nach Anspruch 37, wobei Ar₁ aus der Gruppe, bestehend aus Furan, Thiophen, Pyrrol, Pyrrolin, Pyrrolidin, Oxazol, Thiazol, Imidazol, Imidazolin, Imidazolidin, Pyrazol, Pyrazolin, Pyrazolidin, Isoxazol, Isothiazol, Triazol, Tetrazol, Thiadiazol, Pyran, Pyridin, Piperidin, Morpholin, Thiomorpholin, Pyridazin, Pyrimidin, Pyrazin, Piperazin, Triazin, 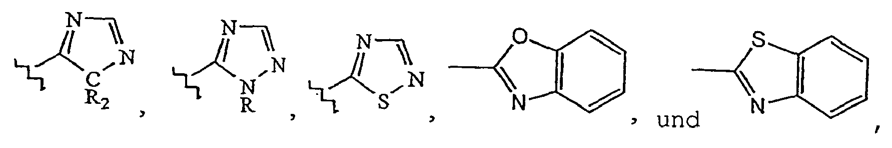 ausgewählt ist.

39. Verbindung nach Anspruch 38, wobei Ar₁ Pyridin, Pyrimidin, 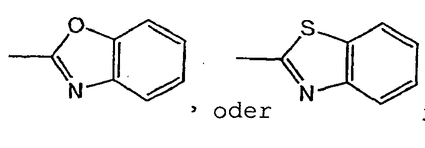 ist.

40. Verbindung nach Anspruch 39, wobei Ar₁ Pyrimidin ist.

41. Verbindung nach einem der Ansprüche 4 und 5, wobei B ein Heteroarylring ist, ausgewählt aus der Gruppe, bestehend aus Furan, Thiophen, Pyrrol, Pyrrolin, Pyrrolidin, Oxazol, Thiazol, Imidazol, Imidazolin, Imidazolidin, Pyrazol, Pyrazolin, Pyrazolidin, Isoxazol, Isothiazol, Triazol, Tetrazol, Thiadiazol, Pyran, Pyridin, Piperidin, Morpholin, Thiomorpholin, Pyridazin, Pyrimidin, Pyrazin, Piperazin, Triazin

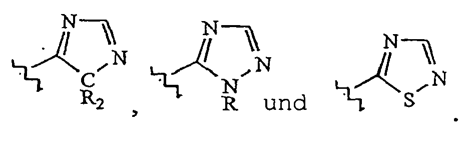

42. Verbindung nach Anspruch 41, wobei B ein Tetrazol ist.

43. Verbindung nach einem der Ansprüche 4 und 5, wobei B - (CH₂)ⱼ-C (O) OR₄ ist.

44. Verbindung nach Anspruch 43, wobei R₄ Wasserstoff oder gegebenenfalls substituiertes Alkyl ist.

45. Verbindung nach Anspruch 44, wobei das Alkyl ein Niederalkyl ist.

46. Verbindung nach Anspruch 45, wobei das Niederalkyl ausgewählt ist aus der Gruppe, bestehend aus Methyl, Ethyl, n-Propyl, Isopropyl, n-Butyl, tert.-Butyl und sec.-Butyl.

47. Verbindung nach einem der Ansprüche 4 und 5, wobei R₄ Wasserstoff ist.

48. Verbindung nach Anspruch 4, die aus der Gruppe, bestehend aus: 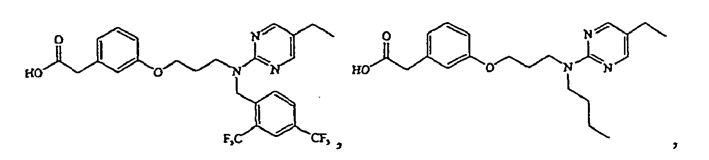 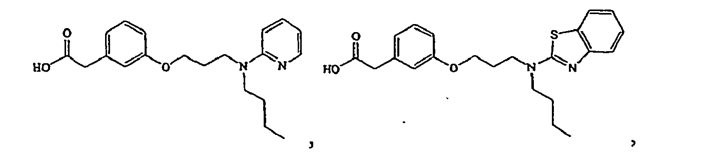 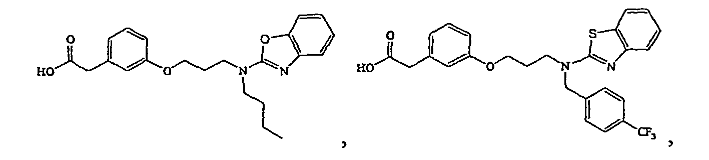 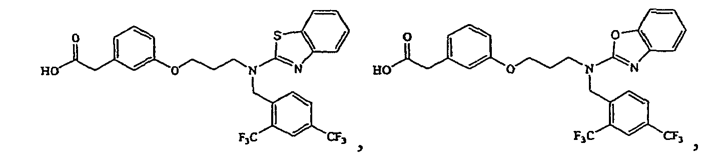 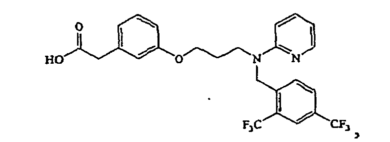 ausgewählt ist, oder ein pharmazeutisch annehmbares N-Oxid, ein pharmazeutisch annehmbares Prodrug, ein pharmazeutisch annehmbarer Metabolit, ein pharmazeutisch annehmbares Salz, ein pharmazeutisch annehmbarer Ester, ein pharmazeutisch annehmbares Amid oder ein pharmazeutisch annehmbares Solvat davon.

49. Verbindung nach Anspruch 5, die aus der Gruppe, bestehend aus 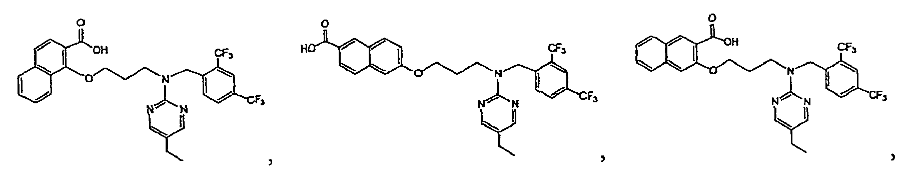 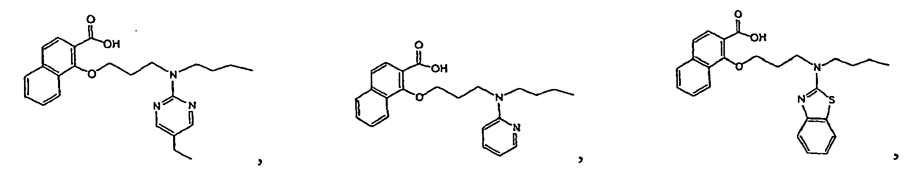 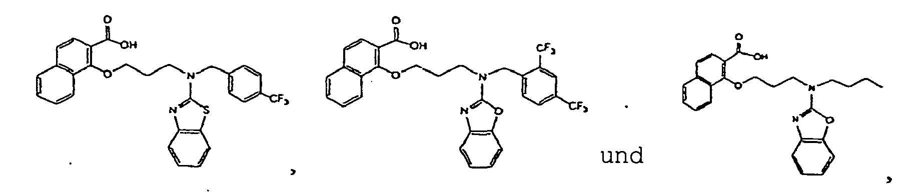 ausgewählt ist, oder ein pharmazeutisch annehmbares N-Oxid, ein pharmazeutisch annehmbares Prodrug, ein pharmazeutisch aktiver Metabolit, ein pharmazeutisch annehmbares Salz, ein pharmazeutisch annehmbarer Ester, ein pharmazeutisch annehmbares Amid oder ein pharmazeutisch annehmbares Solvat davon.

50. Verbindung mit der Struktur der Formel III

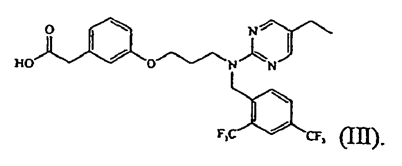

51. Verwendung einer Verbindung nach den Ansprüchen 1 bis 50 zur Herstellung eines Medikaments zur prophylaktischen oder therapeutischen Behandlung einer Peroxisom-Proliferator-aktivierter Rezeptor (PPAR)-modulierten Krankheit oder eines Peroxisom-Proliferator-aktivierter Rezeptor (PPAR)-modulierten Zustands.

52. Verwendung nach Anspruch 51, wobei die Krankheit eine metabolische Störung oder ein metabolischer Zustand ist.

53. Verwendung nach Anspruch 51, wobei die Krankheit ausgewählt ist aus der Gruppe, bestehend aus Fettleibigkeit, Diabetes, Hyperinsulinämie, metabolischem Syndrom X, polycystischem Ovarsyndrom, Klimakterium, mit oxidativem Stress verbundenen Störungen, inflammatorischer Reaktion bis Gewebeschaden, Emphysempathogenese, mit Ischämie verbundenem Organschaden, Doxorubicin-induziertem Herzschaden, Medikamenteninduzierter Hepatotoxizität, Atherosklerose, und hypertoxischem Lungenschaden.

54. Pharmazeutische Zusammensetzung, die eine Verbindung nach den Ansprüchen 1 bis 50 und einen pharmazeutisch annehmbaren Diluenten, Exzipienten oder Träger umfasst.

## Revendications

1. Composé ayant la structure de formule I : 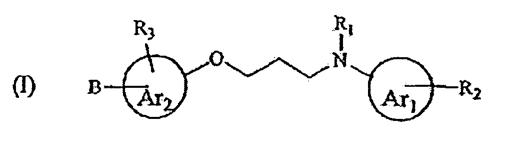 où
Ar₁ est choisi dans le groupe consistant en une structure de cycle hétéroaromatique monocyclique et une structure de cycle hétéroatomique bicyclique ;
Ar₂ est choisi dans le groupe consistant en une structure de cycle aryle carbocyclique monocyclique, bicyclique, et tricyclique
R₁ est choisi dans le groupe consistant en
un groupe alkyle, facultativement substitué par un substituant choisi dans le groupe consistant en un atome d'hydrogène, un groupe alkyle inférieur, un groupe carbocyclique ou hétérocyclique facultativement substitué, un atome d'halogène, un groupe perhalogénoalkyle, hydroxy, alcoxy, nitro et amino ;
un cycle hétéroaryle à cinq chaînons ou à six chaînons ou un cycle aryle à six chaînons, facultativement substitué par un ou plusieurs substituants choisis dans le groupe consistant en un groupe alkyle saturé ou insaturé à chaîne droite, ramifiée ou cyclique en C₁ à C₈ ; alcoxy ; cyano ; nitro ; amino ; amido ; perhalogénoalkyle ; et un atome d'halogène ;
R₂ est choisi dans le groupe consistant en
un atome d'hydrogène ;
un groupe alkyle facultativement substitué par un substituant choisi dans le groupe consistant en un atome d'hydrogène, un groupe alkyle inférieur, un cycle carbocyclique ou hétérocyclique facultativement substitué, un atome d'halogène, un groupe perhalogénoalkyle, hydroxy, alcoxy, nitro et amino ;
un cycle hétéroaryle à cinq chaînons ou six chaînons ou un cycle aryle à six chaînons, facultativement substitué par un ou plusieurs substituants choisis dans le groupe consistant en un groupe alkyle saturé ou insaturé à chaîne droite, ramifiée ou cyclique en C₁ à C₈ facultativement substitué ; alcoxy ; un atome d'halogène ; et un groupe perhalogénoalkyle ;
un groupe cyano ; nitro ; amino ; amido ; perhalogénoalkyle ; et un atome d'halogène ;
R₃ est choisi dans le groupe consistant en un atome d'hydrogène ; un groupe alkyle facultativement substitué par un substituant choisi dans le groupe consistant en un atome d'hydrogène, un groupe alkyle inférieur, un cycle carbocyclique ou hétérocyclique facultativement substitué ; hydroxy ; un atome d'halogène ; un groupe amino ; nitro ; et cyano ; et
B est un cycle hétéroaryle à cinq chaînons ou six chaînons, ou -(CH₂)ⱼ-C(O)OR₄, où j vaut 0 ou 1 lorsque Ar₂ est une structure de cycle carbocyclique, bicyclique ou tricyclique et j vaut 1. lorsque Ar₂ est une structure carbocyclique monocyclique ; et
R₄ est choisi dans le groupe consistant en
un atome d'hydrogène ;
un groupe alkyle facultativement substitué par un substituant choisi dans le groupe consistant en un atome d'hydrogène, un groupe alkyle inférieur, un cycle carbocyclique ou hétérocyclique facultativement substitué ;
un cycle hétéroaryle à cinq chaînons ou à six chaînons ou un cycle aryle à six chaînons, facultativement substitué par un ou plusieurs substituants choisis dans le groupe consistant en un groupe alkyle saturé ou insaturé à chaîne droite, ramifiée ou cyclique en C₁ à C₈ facultativement substitué ;
ou un N-oxyde pharmaceutiquement acceptable, promédicament pharmaceutiquement acceptable, métabolite pharmaceutiquement actif, sel pharmaceutiquement acceptable, ester pharmaceutiquement acceptable, amide pharmaceutiquement acceptable, ou solvate pharmaceutiquement acceptable de celui-ci.

2. Composé selon la revendication 1, où Ar₂ est choisi dans le groupe consistant en un groupe phényle, naphtyle, anthracène, et phénanthrène.

3. Composé selon la revendication 2, où Ar₂ est le phényle.

4. Composé selon la revendication 3, ayant la structure :

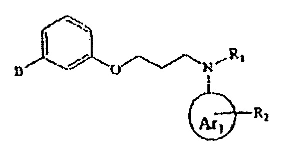

5. Composé selon la revendication 2, où Ar₂ est le naphtyle.

6. Composé selon la revendication 4 ou la revendication 5, où R₁ est un groupe alkyle, facultativement substitué par un ou plusieurs cycles carbocycliques ou hétérocycliques facultativement substitués.

7. Composé selon la revendication 6, où ledit groupe alkyle est un alkyle inférieur.

8. Composé selon la revendication 7, où ledit alkyle inférieur est choisi dans le groupe consistant en le groupe méthyle, éthyle, n-propyle, isopropyle, n-butyle, tert-butyle, et sec-butyle.

9. Composé selon la revendication 6, où ledit cycle carbocyclique est le phényle.

10. Composé selon la revendication 9, où ledit groupe phényle est facultativement substitué par un ou plusieurs substituants choisis dans le groupe consistant en un groupe alkyle inférieur, un atome d'halogène, un groupe perhalogénoalkyle, hydroxy, alxocy, nitro, et amino.

11. Composé selon la revendication 10, où ledit substituant est un groupe perhalogénoalkyle.

12. Composé selon la revendication 11, où ledit groupe perhalogénoalkyle est le trifluorométhyle.

13. Composé selon la revendication 1, où R₁ est substitué par un groupe alkyle avec le 4-bis(trifluorométhyl)phénylméthyle.

14. Composé selon la revendication 1, où Ar₁ est un hétérocycle contenant de l'azote ou contenant de l'oxygène.

15. Composé selon la revendication 14, où Ar₁ est choisi dans le groupe consistant en le furane, le thiophène, le pyrrole, la pyrroline, l'oxazole, le thiazole, l'imidazole, l'imidazoline, l'imidazolidine, le pyrazole, la pyrazoline, la pyrazolidine, l'isoxazole, l'isothiazole, le triazole, le tétrazole, le thiadiazole, le pyrane, la pyridine, la pipéridine, la morpholine, la thiomorpholine, la pyridazine, la pyrimidine, la pyrazine, la pipérazine, la thiazine,

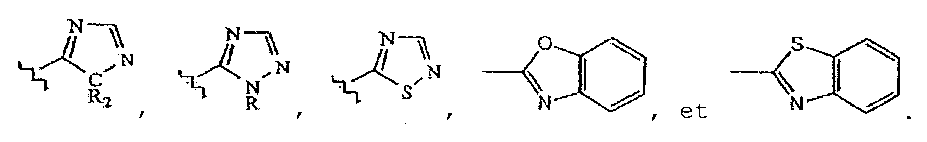

16. Composé selon la revendication 15, où Ar₁ est la pyridine, la pyrimidine,

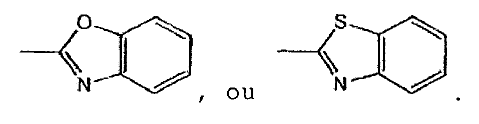

17. Composé selon la revendication 16, où Ar₁ est la pyrimidine.

18. Composé selon l'une quelconque de la revendication 4 et de la revendication 5, où R₂ est facultativement substitué par un groupe alkyle.

19. Composé selon la revendication 18, où ledit groupe alkyle est un alkyle inférieur.

20. Composé selon la revendication 19, dans lequel l'alkyle inférieur est choisi dans le groupe consistant en un groupe méthyle, éthyle, n-propyle, isopropyle, n-butyle, tert-butyle, et sec-butyle.

21. Composé selon la revendication 20, où R₂ est le groupe éthyle.

22. Composé selon la revendication 1, où R₃ est un atome d'hydrogène, d'halogène ou un groupe alkyle facultativement substitué.

23. Composé selon la revendication 22, où ledit groupe alkyle facultativement substitué est un alkyle inférieur facultativement substitué.

24. Composé selon la revendication 23, dans lequel ledit groupe alkyle inférieur facultativement substitué est choisi dans le groupe consistant en le méthyle, l'éthyle, le n-propyle, l'isopropyle, le n-butyle, le tert-butyle, et le sec-butyle.

25. Composé selon la revendication 1, où R₃ est le méthyle.

26. Composé selon la revendication 1, où R₃ est un atome d'hydrogène.

27. Composé selon la revendication 1, où B et les substituants propyloxy sur Ar₂ sont ortho l'un par rapport à l'autre.

28. Composé selon la revendication 1, où B et les substituants propyloxy sur Ar₂ sont méta l'un par rapport à l'autre.

29. Composé selon la revendication 1, où B et les substituants propyloxy sur Ar₂ sont para l'un par rapport à l'autre.

30. Composé selon la revendication 1, où B est un cycle hétéroaryle choisi dans le groupe consistant en le furane, le thiophène, le pyrrole, la pyrroline, l'oxazole, le thiazole, l'imidazole, l'imidazoline, l'imidazolidine, le pyrazole, la pyrazoline, la pyrazolidine, l'isoxazole, l'isothiazole, le triazole, le tétrazole, le thiadiazole, le pyrane, la pyridine, la pipéridine, la morpholine, la thiomorpholine, la pyridazine, la pyrimidine, la pyrazine, la pipérazine, la thiazine,

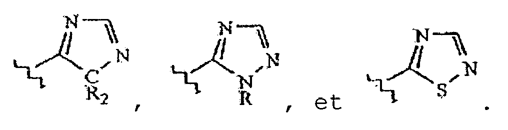

31. Composé selon la revendication 30, où B est un tétrazole.

32. Composé selon la revendication 1, où B est -(CH₂)ⱼ-C(O)OR₄.

33. Composé selon la revendication 32, où R₄ est un atome d'hydrogène ou un groupe alkyle facultativement substitué.

34. Composé selon la revendication 33, où ledit groupe alkyle est un alkyle inférieur.

35. Composé selon la revendication 34, où ledit groupe alkyle inférieur est choisi dans le groupe consistant en le méthyle, éthyle, n-propyle, isopropyle, n-butyle, tert-butyle, et sec-butyle.

36. Composé selon la revendication 33, où R₄ est un atome d'hydrogène.

37. Composé selon l'une quelconque de la revendication 4 et de la revendication 5, où Ar₁ est un hétérocycle contenant de l'azote ou contenant de l'oxygène.

38. Composé selon la revendication 37, où Ar₁ est choisi dans le groupe consistant en le furane, le thiophène, le pyrrole, la pyrroline, l'oxazole, le thiazole, l'imidazole, l'imidazoline, l'imidazolidine, le pyrazole, la pyrazoline, la pyrazolidine, l'isoxazole, l'isothiazole, le triazole, le tétrazole, le thiadiazole, le pyrane, la pyridine, la pipéridine, la morpholine, la thiomorpholine, la pyridazine, la pyrimidine, la pyrazine, la pipérazine, la thiazine,

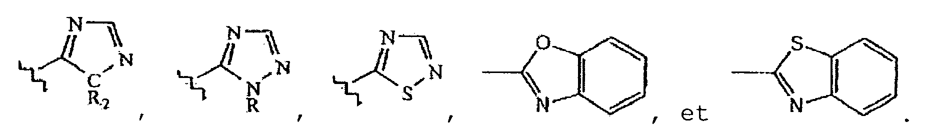

39. Composé selon la revendication 38, où Ar₁ est la pyridine, la pyrimidine,

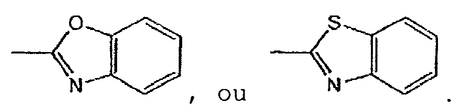

40. Composé selon la revendication 39, où Ar₁ est la pyrimidine.

41. Composé selon l'une quelconque de la revendication 4 et de la revendication 5, où B est un cycle hétéroaryle choisi dans le groupe consistant en le furane, le thiophène, le pyrrole, la pyrroline, l'oxazole, le thiazole, l'imidazole, l'imidazoline, l'imidazolidine, le pyrazole, la pyrazoline, la pyrazolidine, l'isoxazole, l'isothiazole, le triazole, le tétrazole, le thiadiazole, le pyrane, la pyridine, la pipéridine, la morpholine, la thiomorpholine, la pyridazine, la pyrimidine, la pyrazine, la pipérazine, la thiazine,

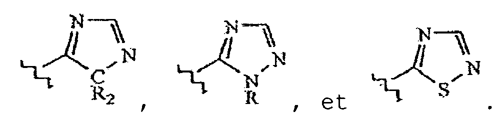

42. Composé selon la revendication 41, où B est un tétrazole.

43. Composé selon l'une quelconque de la revendication 4 et de la revendication 5, où B est -(CH₂)ⱼ-C(O)OR₄.

44. Composé selon la revendication 43, où R₄ est un atome d'hydrogène ou un groupe alkyle facultativement substitué.

45. Composé selon la revendication 44, où ledit groupe alkyle est un alkyle inférieur.

46. Composé selon la revendication 45, où ledit groupe alkyle inférieur est choisi dans le groupe consistant en le méthyle, l'éthyle, le n-propyle, l'isopropyle, le n-butyle, le tert-butyle, et le sec-butyle.

47. Composé selon l'une quelconque de la revendication 4 et de la revendication 5, où R₄ est un atome d'hydrogène.

48. Composé selon la revendication 4, choisi dans le groupe consistant en : 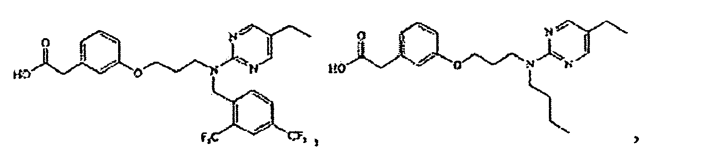 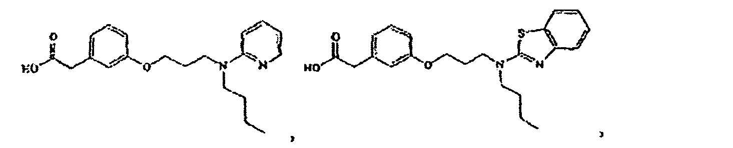 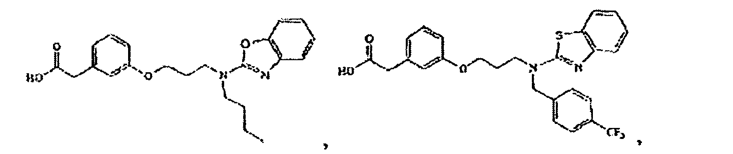 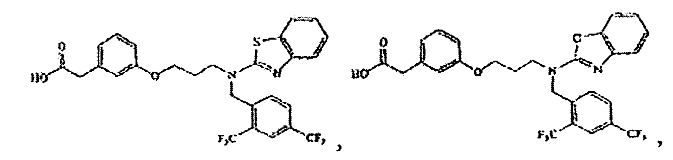 et 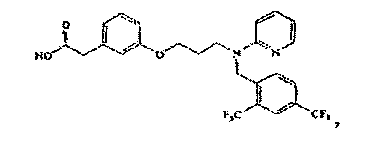 ou un N-oxyde pharmaceutiquement acceptable, un promédicament pharmaceutiquement acceptable, un métabolite pharmaceutiquement actif, un sel pharmaceutiquement acceptable, un ester pharmaceutiquement acceptable, un amide pharmaceutiquement acceptable, ou un solvate pharmaceutiquement acceptable.

49. Composé selon la revendication 5 choisi dans le groupe consistant en 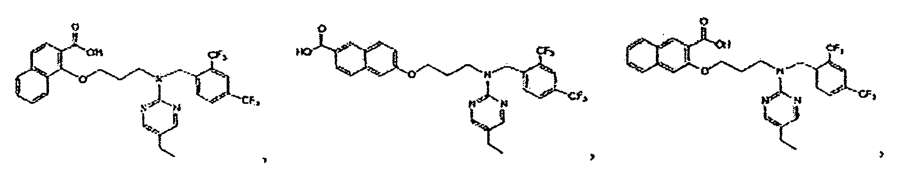 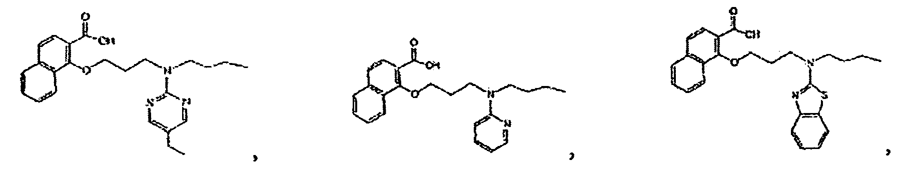 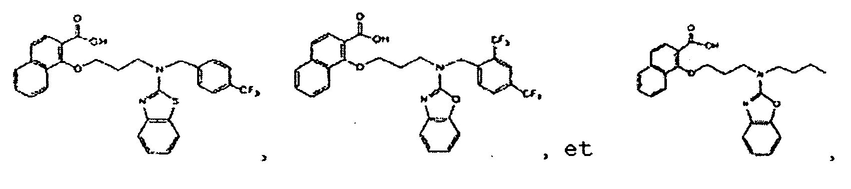 ou un N-oxyde pharmaceutiquement acceptable, un promédicament pharmaceutiquement acceptable, un métabolite pharmaceutiquement actif, un sel pharmaceutiquement acceptable, un ester pharmaceutiquement acceptable, un amide pharmaceutiquement acceptable, ou un solvate pharmaceutiquement acceptable.

50. Composé ayant la structure de la formule III :

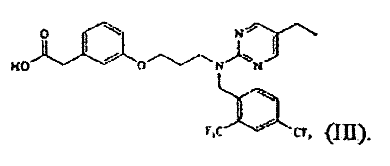

51. Utilisation d'un composé comme revendiqué dans les revendications 1 à 50 pour la préparation d'un médicament destiné au traitement prophylactique ou thérapeutique d'une maladie ou affection modulée par récepteur activé par proliférateur de péroxysome (RAPP).

52. Utilisation selon la revendication 51, où la maladie est un trouble ou affection métabolique.

53. Utilisation selon la revendication 51, où ladite maladie est choisie dans le groupe consistant en l'obésité, le diabète, l'hyperinsulinisme, le syndrome métabolique X, le syndrome polycystique ovarien, le climatère, les troubles associés au stress oxydatif, la réponse inflammatoire à une lésion du tissu, la pathogénie de l'emphysème, une lésion d'organe associée à l'ischémie, une lésion cardiaque induite par doxorubicine, l'hépatoxicité induite par médicament, l'athérosclérose, et une lésion hypertoxique du poumon.

54. Composition pharmaceutique comprenant un composé des revendications 1 à 50 et un diluant, excipient, ou véhicule pharmaceutiquement acceptable.
